# EUROPEAN PATENT SPECIFICATION

(11) **EP 3 166 502 B1**
(45) Date of publication and mention of the grant of the patent: **16.04.2025**
(21) Application number: 15818502.5
(22) Date of filing: 08.07.2015
(51) Int. Cl.: A61B 8/13, A61B 8/00, H01R 13/631, H01R 107/00, H01R 12/88

(54) **MODULAR AND PORTABLE ULTRASOUND SYSTEMS AND METHODS**
MODULARE UND TRAGBARE ULTRASCHALLSYSTEME UND VERFAHREN
SYSTÈMES À ULTRASONS PORTABLES ET MODULAIRES ET PROCÉDÉS

(30) Priority: 09.07.2014 US 201462022603 P
(43) Date of publication of application: 17.05.2017
(73) Proprietor: Edan Instruments, Inc., Nanshan, Shenzhen 518067 (CN)
(72) Inventor: MURPHY, Sean, Sunnyvale, California 94089 (US); HENDERSON, Richard, Sunnyvale, California 94089 (US)
(74) Representative: Herrero & Asociados, S.L.
(86) International application number: PCT/US2015/039602
(87) International publication number: WO 2016/007667

(56) References cited:
- WO-A1-2014/041503
- US-A- 5 328 381
- US-A- 5 692 208
- US-A- 5 692 208
- US-A1- 2002 082 479
- US-A1- 2003 158 482
- US-A1- 2004 152 982
- US-A1- 2004 152 982
- US-A1- 2005 251 035
- US-A1- 2008 055 826
- US-A1- 2011 055 447
- US-B1- 6 261 130
- US-B1- 7 549 961

## Description

### BACKGROUND

The present disclosure relates generally to the field of portable ultrasound devices. Ultrasound devices image a patient by producing and emitting ultrasonic waves with a transducer. The transducer measures returning echoes of these waves to provide data regarding the patient. The data may be analyzed and assembled into an image of the patient using a computing device. Typically, portable ultrasound devices are large systems transported on a cart with limited battery life. Alternatively, some portable ultrasound systems are hand held but still relatively large.

For example, US20040152982A1 discloses a modular diagnostic ultrasound apparatus comprising a core unit, system electronics and an I/O port. The core unit comprises a housing and a system electronics package within the housing. The system electronics having one or more concatenated filters, including a front end transmit/receive circuit, a processor, a back end circuit for scan conversion, a system clock and a programmable system memory device. There is at least one I/O port connected to the front end and the back end of the system electronics package and extending through the core unit housing wherein all system data processing information is transmitted or received through the I/O port.

For example, US5328381A discloses an electrical and electronic connector module having six degrees of freedom. The connector module is particularly useful in advanced electrical and electronic wiring systems, such as multiplexed wiring systems, wherein many electrical functions or signals share common wire pathways. The connector module comprises a male header containing a plurality of male connector elements. A plurality of female connector elements disposed in a receptacle assembly is designed to align and mate with respective ones of the male connector elements disposed in the male header. The header and receptacle align by means of a pair of header shoulder bolts that seat within apertures disposed on distal ends of the receptacle. The mating of the male-female connectors is assured by reason that the receptacle is allowed to float within a housing, which forms part of the receptacle assembly.

For example, US5692208A discloses a lever apparatus for use in a personal computer that has a chassis with a wall and an interior portion that contains a frame configured to retain an ejectable structure therein. The frame has an ejector mechanism associated therewith for ejecting an ejectable structure therefrom, and the lever apparatus actuates the ejector mechanism to eject the ejectable structure from the frame. The lever apparatus comprises an elongated body pivotally that is coupled to the wall and that has an axis of rotation with respect to the wall. The elongated body may be pivotally coupled to the wall by a pin and hinge member The elongated body has a digit contact end external to the interior portion and an oppositely disposed cam end operable against the ejector mechanism. The cam end exerts a leverage force against the ejector mechanism when the elongated body is pivoted from a non-engaged position to an engaged position, to thereby eject the ejectable structure from the frame.

The present disclosure includes features which enhance the portability, usability, and configurability or portable ultrasound system.

### SUMMARY

The present disclosure provides a portable ultrasound system, a removable ultrasound module for the portable ultrasound system, and a system. The portable ultrasound system, the removable ultrasound module for the portable ultrasound system, and the system are as set out claims 1-10.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

FIG. 1 illustrates an embodiment of a portable ultrasound system incorporating aspects of the present disclosure.
FIG. 2 illustrates a front view of one embodiment of a portable ultrasound system.
FIG. 3 illustrates a block diagram of components of one embodiment of a portable ultrasound system.
FIG. 4 is an illustration of an exemplary embodiment of an ultrasound module.
FIG. 5 is an illustration of an exemplary embodiment of a power supply module.
FIG. 6 is an illustration of an exemplary embodiment of a main circuit board module.
FIG. 7A is an illustration of an exemplary layout of an ultrasound module, power supply module, and main circuit board module when connected.
FIG. 7B is an illustration of modules of a portable ultrasound system in relation to a housing and frame of the system according to an exemplary embodiment.
FIG. 8 illustrates an exemplary embodiment of a portable ultrasound system with an ultrasound module removed or not connected.
FIG. 9 illustrates an exemplary embodiment of an access door housing a release lever for disconnecting an ultrasound module.
FIG. 10A illustrates a release lever access door according to an exemplary embodiment.
FIG. 10B illustrates a release lever access door being opened to provide access to a release lever according to an exemplary embodiment.
FIG. 10C illustrates an exposed release lever according to an exemplary embodiment.
FIG. 10D illustrates a release lever being actuated according to an exemplary embodiment.
FIG. 10E illustrates an actuated release lever and a disconnected ultrasound module according to an exemplary embodiment.
FIG. 11 is an illustration of a lower portion of a release lever in relation to a main housing and an ultrasound module according to an exemplary embodiment.
FIG. 12 is an illustration of an exemplary embodiment of a release lever and some of the components thereof.
FIG. 13 illustrates an exemplary embodiment of a frame, main housing, and release lever.
FIG. 14 illustrates an exemplary embodiment of a frame of a portable ultrasound system.
FIG. 15 illustrates an exemplary embodiment of a frame of a portable ultrasound system supporting and/or guiding an ultrasound module.
FIG. 16 illustrates an exemplary embodiment of a frame in relation to an inserted ultrasound module.
FIG. 17 illustrates an exemplary embodiment of an ultrasound module and connectors for connecting to other components of portable ultrasound system.
FIG. 18 illustrates an exemplary embodiment of a female fixed connector of an ultrasound module.
FIG. 19 illustrates an exemplary embodiment of a male fixed connector of a main circuit board module.
FIG. 20 illustrates an exemplary embodiment of male floating connector of an ultrasound module.
FIG. 21 illustrates an exemplary embodiment of a female floating connector of a power supply module.
FIG. 22 is an illustration of an ultrasound module including heat sinks according to an exemplary embodiment.
FIG. 23 is an illustration of a heat shield in relation to a touchscreen and touchpad of a portable ultrasound system according to an exemplary embodiment.
FIG. 24 illustrates an exemplary embodiment of a heat shield in relationship to a frame of a portable ultrasound system.
FIG. 25 illustrates an exemplary embodiment of a heat shield of portable ultrasound system.
FIG. 26 illustrates an exemplary embodiment of a heat shield in relationship to an ultrasound module.

### DETAILED DESCRIPTION OF THE PREFERRED EMBODIMENTS

Generally, the present disclosure relates to features for a portable ultrasound system. The features enhance the portability, configurability, and functionality of the portable ultrasound system. A portable ultrasound system is typically battery powered. The system may also be powered by mains power when available. The portable ultrasound system may be used for obstetrical and gynecological imaging (e.g., measuring the size of a fetus, checking the position of a fetus, etc.), cardiac imaging (e.g., identifying abnormal heart structures, measuring blood flow, etc.), urological imaging, etc. As portable ultrasound systems may be used in less than ideal conditions (e.g., no ready access to power, no formal work station, etc.), the features described herein help to address the problems associated with such use.

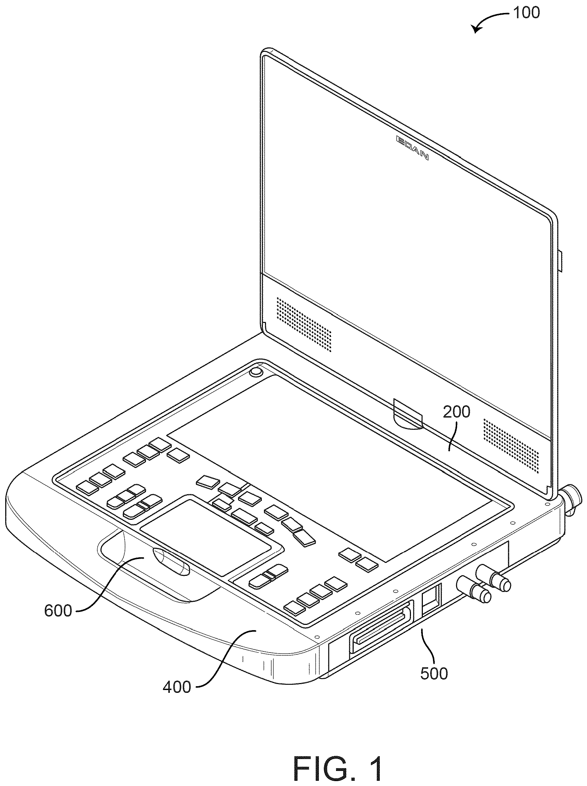

Referring to FIG. 1, one embodiment of portable ultrasound system 100 is illustrated. Portable ultrasound system 100 may include display support system 200 for increasing the durability of the display system. Portable ultrasound system 100 may further include locking lever system 500 for securing ultrasound probes and/or transducers. Some embodiments of portable ultrasound system 100 include ergonomic handle system 400 for increasing portability and usability. Further embodiments include status indicator system 600 which displays, to a user, information relevant to portable ultrasound system 100. Portable ultrasound system 100 may further include features such as an easy to operate and customizable user interface, adjustable feet, a backup battery, modular construction, cooling systems, etc.

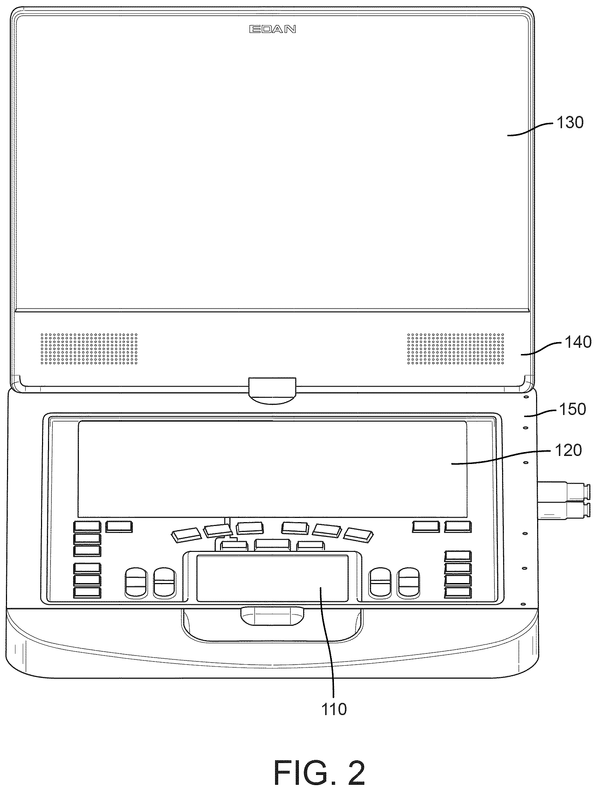

Referring to FIG. 2, a front view of one embodiment of portable ultrasound system 100 is illustrated. Main housing 150 houses components of portable ultrasound system 100. In some embodiments, the components housed within main housing 150 include locking lever system 500, ergonomic handle system 400, and status indicator system 600. Main housing 150 may also be configured to support electronics modules which may be replaced and/or upgraded due to the modular construction of portable ultrasound system 100. In some embodiments, portable ultrasound system 100 includes display housing 140. Display housing 140 may include display support system 200. In some embodiments, portable ultrasound system 100 includes touchpad 110 for receiving user inputs and displaying information, touchscreen 120 for receiving user inputs and displaying information, and main screen 130 for displaying information.

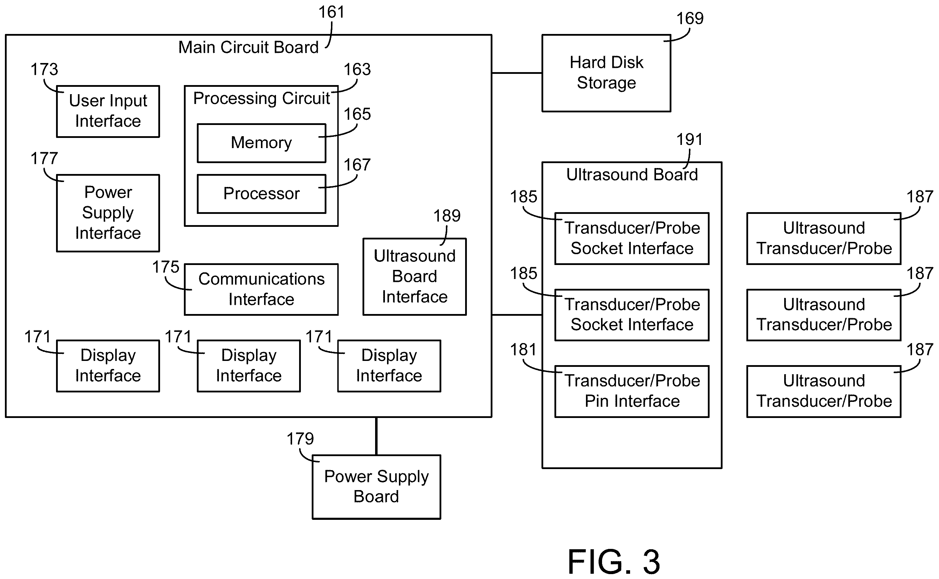

Referring to FIG. 3, a block diagram shows internal components of one embodiment of portable ultrasound system 100. Portable ultrasound system 100 includes main circuit board 161. Main circuit board 161 carries out computing tasks to support the functions of portable ultrasound system 100 and provides connection and communication between various components of portable ultrasound system 100. In some embodiments, main circuit board 161 is configured so as to be a replaceable and/or upgradable module.

To perform computational, control, and/or communication tasks, main circuit board 161 includes processing circuit 163. Processing circuit 163 is configured to perform general processing and to perform processing and computational tasks associated with specific functions of portable ultrasound system 100. For example, processing circuit 163 may perform calculations and/or operations related to producing an image from signals and or data provided by ultrasound equipment, running an operating system for portable ultrasound system 100, receiving user inputs, etc. Processing circuit 163 may include memory 165 and processor 167 for use in processing tasks. For example, processing circuit may perform calculations and/or operations.

Processor 167 may be, or may include, one or more microprocessors, application specific integrated circuits (ASICs), circuits containing one or more processing components, a group of distributed processing components, circuitry for supporting a microprocessor, or other hardware configured for processing. Processor 167 is configured to execute computer code. The computer code may be stored in memory 165 to complete and facilitate the activities described herein with respect to portable ultrasound system 100. In other embodiments, the computer code may be retrieved and provided to processor 167 from hard disk storage 169 or communications interface 175 (e.g., the computer code may be provided from a source external to main circuit board 161).

Memory 165 can be any volatile or non-volatile computer-readable storage medium capable of storing data or computer code relating to the activities described herein. For example, memory 165 may include modules which are computer code modules (e.g., executable code, object code, source code, script code, machine code, etc.) configured for execution by processor 167. Memory 165 may include computer executable code related to functions including ultrasound imagining, battery management, handling user inputs, displaying data, transmitting and receiving data using a wireless communication device, etc. In some embodiments, processing circuit 163 may represent a collection of multiple processing devices (e.g., multiple processors, etc.). In such cases, processor 167 represents the collective processors of the devices and memory 165 represents the collective storage devices of the devices. When executed by processor 167, processing circuit 163 is configured to complete the activities described herein as associated with portable ultrasound system 100.

Hard disk storage 169 may be a part of memory 165 and/or used for non-volatile long term storage in portable ultrasound system 100. Hard disk storage 169 may store local files, temporary files, ultrasound images, patient data, an operating system, executable code, and any other data for supporting the activities of portable ultrasound device 100 described herein. In some embodiments, hard disk storage is embedded on main circuit board 161. In other embodiments, hard disk storage 169 is located remote from main circuit board 161 and coupled thereto to allow for the transfer of data, electrical power, and/or control signals. Hard disk 169 may be an optical drive, magnetic drive, a solid state hard drive, flash memory, etc.

In some embodiments, main circuit board 161 includes communications interface 175. Communications interface 175 may include connections which enable communication between components of main circuit board 161 and communications hardware. For example, communications interface 175 may provide a connection between main circuit board 161 and a network device (e.g., a network card, a wireless transmitter/receiver, etc.). In further embodiments, communications interface 175 may include additional circuitry to support the functionality of attached communications hardware or to facilitate the transfer of data between communications hardware and main circuit board 161. In other embodiments, communications interface 175 may be a system on a chip (SOC) or other integrated system which allows for transmission of data and reception of data. In such a case, communications interface 175 may be coupled directly to main circuit board 161 as either a removable package or embedded package.

Some embodiments of portable ultrasound system 100 include power supply board 179. Power supply board 179 includes components and circuitry for delivering power to components and devices within and/or attached to portable ultrasound system 100. In some embodiments, power supply board 179 includes components for alternating current and direct current conversion, for transforming voltage, for delivering a steady power supply, etc. These components may include transformers, capacitors, modulators, etc. to perform the above functions. In further embodiments, power supply board 179 includes circuitry for determining the available power of a battery power source. In other embodiments, power supply board 179 may receive information regarding the available power of a battery power source from circuitry located remote from power supply board 179. For example, this circuitry may be included within a battery. In some embodiments, power supply board 179 includes circuitry for switching between power sources. For example, power supply board 179 may draw power from a backup battery while a main battery is switched. In further embodiments, power supply board 179 includes circuitry to operate as an uninterruptable power supply in conjunction with a backup battery. Power supply board 179 also includes a connection to main circuit board 161. This connection may allow power supply board 179 to send and receive information from main circuit board 161. For example, power supply board 179 may send information to main circuit board 161 allowing for the determination of remaining battery power. The connection to main circuit board 161 may also allow main circuit board 161 to send commands to power supply board 179. For example, main circuit board 161 may send a command to power supply board 179 to switch from source of power to another (e.g., to switch to a backup battery while a main battery is switched). In some embodiments, power supply board 179 is configured to be a module. In such cases, power supply board 179 may be configured so as to be a replaceable and/or upgradable module. In some embodiments, power supply board 179 is or includes a power supply unit. The power supply unit may convert AC power to DC power for use in portable ultrasound system 100. The power supply may perform additional functions such as short circuit protection, overload protection, undervoltage protection, etc. The power supply may conform to ATX specification. In other embodiments, one or more of the above described functions may be carried out by main circuit board 161.

Main circuit board 161 may also include power supply interface 177 which facilitates the above described communication between power supply board 179 and main circuit board 161. Power supply interface 177 may include connections which enable communication between components of main circuit board 161 and power supply board 179. In further embodiments, power supply interface 177 includes additional circuitry to support the functionality of power supply board 179. For example, power supply interface 177 may include circuitry to facilitate the calculation of remaining battery power, manage switching between available power sources, etc. In other embodiments, the above described functions of power supply board 179 may be carried out by power supply interface 177. For example, power supply interface 177 may be a SOC or other integrated system. In such a case, power supply interface 177 may be coupled directly to main circuit board 161 as either a removable package or embedded package.

With continued reference to FIG. 3, some embodiments of main circuit board 161 include user input interface 173. User input interface 173 may include connections which enable communication between components of main circuit board 161 and user input device hardware. For example, user input interface 173 may provide a connection between main circuit board 161 and a capacitive touchscreen, resistive touchscreen, mouse, keyboard, buttons, and/or a controller for the proceeding. In one embodiment, user input interface 173 couples controllers for touchpad 110, touchscreen 120, and main screen 130 to main circuit board 161. In other embodiments, user input interface 173 includes controller circuitry for touchpad 110, touchscreen 120, and main screen 130. In some embodiments, main circuit board 161 includes a plurality of user input interfaces 173. For example, each user input interface 173 may be associated with a single input device (e.g., touchpad 110, touchscreen 120, a keyboard, buttons, etc.).

**In** further embodiments, user input interface 173 may include additional circuitry to support the functionality of attached user input hardware or to facilitate the transfer of data between user input hardware and main circuit board 161. For example, user input interface 173 may include controller circuitry so as to function as a touchscreen controller. User input interface 173 may also include circuitry for controlling haptic feedback devices associated with user input hardware. In other embodiments, user input interface 173 may be a SOC or other integrated system which allows for receiving user inputs or otherwise controlling user input hardware. In such a case, user input interface 173 may be coupled directly to main circuit board 161 as either a removable package or embedded package.

Main circuit board 161 may also include ultrasound board interface 189 which facilitates communication between ultrasound board 179 and main circuit board 161. Ultrasound board interface 189 may include connections which enable communication between components of main circuit board 161 and ultrasound board 191. In further embodiments, ultrasound board interface 189 includes additional circuitry to support the functionality of ultrasound board 191. For example, ultrasound board interface 189 may include circuitry to facilitate the calculation of parameters used in generating an image from ultrasound data provided by ultrasound board 191. In some embodiments, ultrasound board interface 189 is a SOC or other integrated system. In such a case, ultrasound board interface 189 may be coupled directly to main circuit board 161 as either a removable package or embedded package.

**In** other embodiments, ultrasound board interface 189 includes connections which facilitate use of a modular ultrasound board 191. Ultrasound board 191 may be a module (e.g., ultrasound module) capable of performing functions related to ultrasound imaging (e.g., multiplexing sensor signals from an ultrasound probe/transducer, controlling the frequency of ultrasonic waves produced by an ultrasound probe/transducer, etc.). The connections of ultrasound board interface 189 may facilitate replacement of ultrasound board 191 (e.g., to replace ultrasound board 191 with an upgraded board or a board for a different application). For example, ultrasound board interface 189 may include connections which assist in accurately aligning ultrasound board 191 and/or reducing the likelihood of damage to ultrasound board 191 during removal and or attachment (e.g., by reducing the force required to connect and/or remove the board, by assisting, with a mechanical advantage, the connection and/or removal of the board, etc.).

In embodiments of portable ultrasound system 100 including ultrasound board 191, ultrasound board 191 includes components and circuitry for supporting ultrasound imaging functions of portable ultrasound system 100. In some embodiments, ultrasound board 191 includes integrated circuits, processors, and memory. Ultrasound board 191 may also include one or more transducer/probe socket interfaces 185. Transducer/probe socket interface 185 enables ultrasound transducer/probe 187 (e.g., a probe with a socket type connector) to interface with ultrasound board 191. For example, transducer/probe socket interface 185 may include circuitry and/or hardware connecting ultrasound transducer/probe 187 to ultrasound board 191 for the transfer of electrical power and/or data. Transducer/probe socket interface 185 may include hardware which locks ultrasound transducer/probe 187 into place (e.g., a slot which accepts a pin on ultrasound transducer/probe 187 when ultrasound transducer/probe 187 is rotated). In some embodiments, ultrasound board 191 includes two transducer/probe socket interfaces 185 to allow the connection of two socket type ultrasound transducers/probes 187.

In some embodiments, ultrasound board 191 also includes one or more transducer/probe pin interfaces 181. Transducer/probe pin interface 181 enables an ultrasound transducer/probe 187 with a pin type connector to interface with ultrasound board 191. Transducer/probe pin interface 181 may include circuitry and/or hardware connecting ultrasound transducer/probe 187 to ultrasound board 191 for the transfer of electrical power and/or data. Transducer/probe pin interface 181 may include hardware which locks ultrasound transducer/probe 187 into place. In some embodiments, ultrasound transducer/probe 187 is locked into place with locking lever system 500. In some embodiments, ultrasound board 191 includes more than one transducer/probe pin interfaces 181 to allow the connection of two or more pin type ultrasound transducers/probes 187. In such cases, portable ultrasound system 100 may include one or more locking lever systems 500. In further embodiments, ultrasound board 191 may include interfaces for additional types of transducer/probe connections.

With continued reference to FIG. 3, some embodiments of main circuit board 161 include display interface 171. Display interface 171 may include connections which enable communication between components of main circuit board 161 and display device hardware. For example, display interface 171 may provide a connection between main circuit board 161 and a liquid crystal display, a plasma display, a cathode ray tube display, a light emitting diode display, and/or a display controller or graphics processing unit for the proceeding or other types of display hardware. In some embodiments, the connection of display hardware to main circuit board 161 by display interface 171 allows a processor or dedicated graphics processing unit on main circuit board 161 to control and/or send data to display hardware. Display interface 171 may be configured to send display data to display device hardware in order to produce an image. In some embodiments, main circuit board 161 includes multiple display interfaces 171 for multiple display devices (e.g., three display interfaces 171 connect three displays to main circuit board 161). In other embodiments, one display interface 171 may connect and/or support multiple displays. In one embodiment, three display interfaces 171 couple touchpad 110, touchscreen 120, and main screen 130 to main circuit board 161.

In further embodiments, display interface 171 may include additional circuitry to support the functionality of attached display hardware or to facilitate the transfer of data between display hardware and main circuit board 161. For example, display interface 171 may include controller circuitry, a graphics processing unit, video display controller, etc. In some embodiments, display interface 171 may be a SOC or other integrated system which allows for displaying images with display hardware or otherwise controlling display hardware. Display interface 171 may be coupled directly to main circuit board 161 as either a removable package or embedded package. Processing circuit 163 in conjunction with one or more display interfaces 171 may display images on one or more of touchpad 110, touchscreen, 120, and main screen 130.

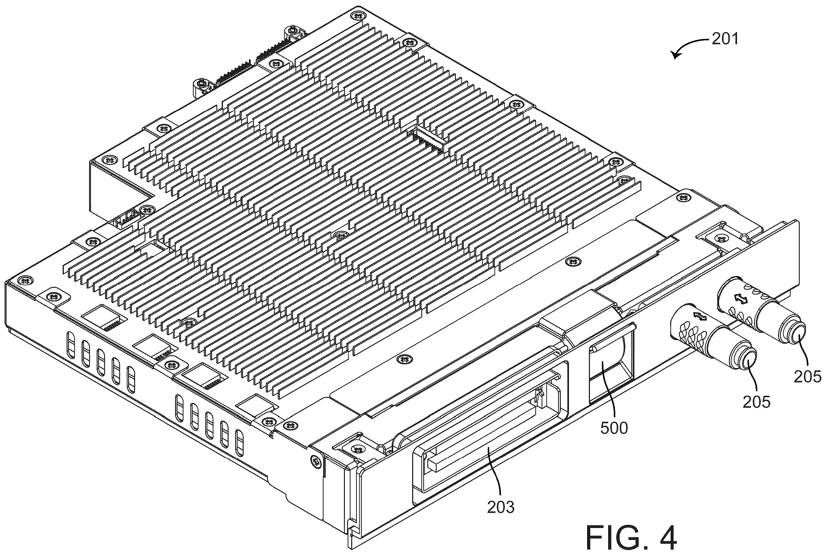

Referring to FIG. 4, an exemplary embodiment of an ultrasound module 201 according to the present disclosure is illustrated. In some embodiments, ultrasound module 201 houses the ultrasound components of portable ultrasound system 100. The ultrasound components of portable ultrasound system 100 may be housed entirely or partially within ultrasound module 201.

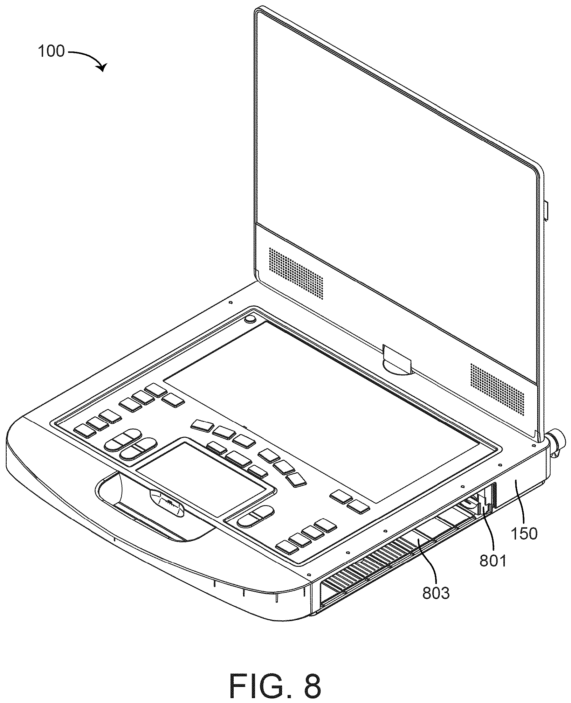

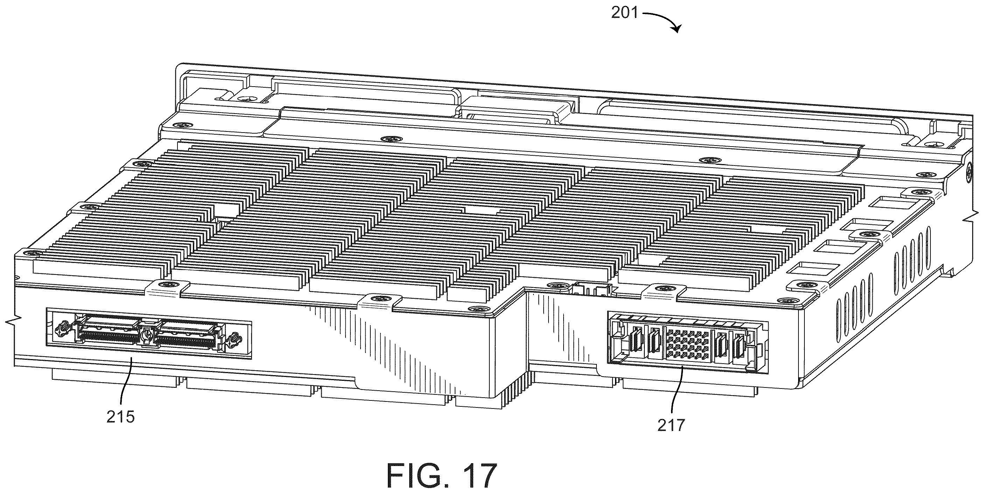

Advantageously, ultrasound module 201 may be configured to be removable from portable ultrasound system 100 as discussed later and in more detail with reference to FIGS. 8- 16 herein. This function may be supported by connectors facilitating the connection and disconnection of ultrasound module 201 as in more detail with reference to FIGS. 17-21. This allows portable ultrasound system 100 to accept different ultrasound modules 201. Ultrasound modules 201 with varying specifications may be inserted into portable ultrasound system 100. For example, an ultrasound module 201 with hardware targeted at specific clinical applications may be substituted or upgraded for an ultrasound module 201 dedicated to an expanded range of application. Advantageously, swapping in and out specialized ultrasound modules 201 allows the overall package of portable ultrasound system 100 to be smaller and lighter without sacrificing performance for various tasks. Additionally, the reconfigurable and module nature of portable ultrasound system 100 allows a user to customize portable ultrasound system 100 (e.g., through swapping ultrasound modules 201). Additionally, the module nature of portable ultrasound system 100 provides an advantage of allowing a user to upgrade the ultrasound performance of the system by replacing an ultrasound module 201 with a different (e.g., more powerful, accurate, sophisticated, etc.) ultrasound module. A damaged ultrasound module (e.g., damaged by harsh operating conditions encountered by portable ultrasound system 100) may be replaced easily due to the module nature of the ultrasound module 201.

Ultrasound module 201 may include input interfaces for ultrasound equipment. In some embodiments, ultrasound module 201 includes one or more pin type ultrasound probe interfaces 203. Pin type ultrasound interface 203 may allow an ultrasound probe to connect to an ultrasound board 191 included in ultrasound module 201. For example, an ultrasound probe connected to pin type ultrasound interface 203 may be connected to ultrasound board 191 via transducer/probe pin interface 181. In some embodiments, pin type ultrasound interface 203 allows communication between components of portable ultrasound system 100 (e.g., ultrasound module 201, power supply module 301, main circuit board module 401, or other components included in or connected with portable ultrasound system 100) and an ultrasound probe. For example, control signals may be provided to an ultrasound probe (e.g., controlling the ultrasound emissions of the probe) and data may be received by ultrasound module 201 from the probe (e.g., imaging data).

In some embodiments, ultrasound module 201 may include locking lever system 500 for securing an ultrasound probe. For example, an ultrasound probe may be secured in pin type ultrasound probe interface 203 by locking lever system 500.

In further embodiments, ultrasound module 201 includes one or more socket type ultrasound probe interfaces 205. Socket type ultrasound probe interfaces 205 may allow a socket type ultrasound probe to connect to an ultrasound board 191 included in ultrasound module 201. For example, an ultrasound probe connected to socket type ultrasound probe interface 205 may be connected to ultrasound board 191 via transducer/probe socket interface 185. In some embodiments, socket type ultrasound probe interface 205 allows communication between components of portable ultrasound system 100 (e.g., ultrasound module 201, power supply module 301, main circuit board module 401, or other components included in or connected with portable ultrasound system 100). For example, control signals may be provided to an ultrasound probe (e.g., controlling the ultrasound emissions of the probe) and data may be received by ultrasound module 201 from the probe (e.g., imaging data).

In further embodiments, ultrasound module 201 includes computation, control, or other support hardware. The support hardware may facilitate the ultrasound functions performed by ultrasound module 201. For example, ultrasound module 201 may include processors, memory, integrated circuits, graphics processing units (GPU), central processing units (CPU), controllers, application specific integrated circuits (ASICs), or other computational hardware. Ultrasound module 201 may perform tasks such as controlling ultrasound probe output (e.g., controlling active transducer elements, beam steering, controlling ultrasound pulses, etc.), receiving ultrasound probe input, performing post-processing, generating images from input provided by an ultrasound probe, storing input data, storing images, or otherwise manipulating data and/or hardware related to ultrasound imaging.

**In** some embodiments, main circuit board 161 and/or power supply board 179 are configured to support ultrasound modules 201 using typically 128 channels or 64 channels for imaging but not limited to either 128 or 64 channel configurations. Advantageously, this allows for a user of the system to perform imaging tasks with a less expensive channel count ultrasound module 201 but switch to a higher channel count ultrasound module 201 when desired (e.g., to achieve greater resolution) easily and quickly due to the modular nature of portable ultrasound system 100. In some embodiments, the connections between ultrasound module 201 and other components is configured to facilitate this operation. For example, connectors may designed to accept multiple configurations of connectors on ultrasound module 201 (e.g., a 64 channel ultrasound module 201 may have fewer pins or other connection features than a 128 channel ultrasound module 201). In other embodiments, the components of portable ultrasound system 100 and/or ultrasound module 201 may determine the number of channels used by a connected ultrasound module 201 and adjust parameters accordingly. For example, power provided to ultrasound module 201 may be increased or decreased, cooling fan speed may be increased or decreased, more or less memory may be allocated to systems of ultrasound module 201, a user interface may be adjusted to display more, less, or different information, and/or other parameters of components or functions of portable ultrasound system 100 may be adjusted.

Ultrasound module 201 can connect to one or more of the other components of portable ultrasound system 100 (e.g., power supply module 301 and main circuit board module 401) by connectors configured to support the modularity of the ultrasound module 201. For example, the connectors may be configured to ensure accurate and complete connection between ultrasound module 201 and other components while still allowing for the removal of ultrasound module 201. The connectors are discussed with greater detail later and with reference to FIGS. 17-21 herein. When connected to other components of portable ultrasound system 100, ultrasound module 201 may be housed within main housing 150 of portable ultrasound system 100 as depicted in FIGS. 1 and 2.

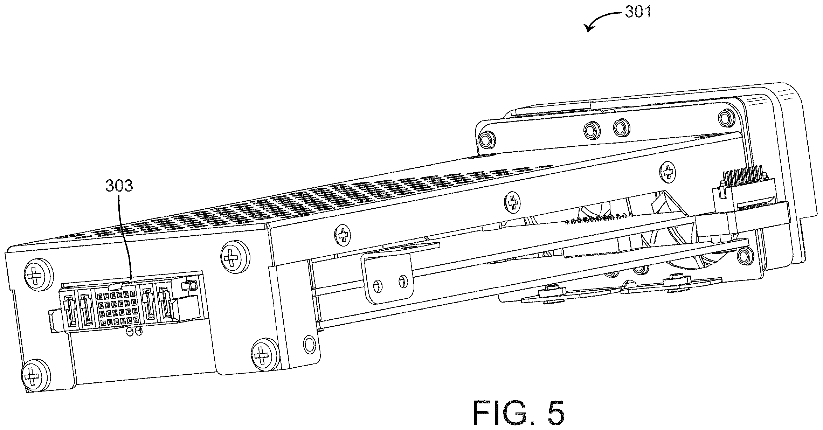

Referring now to FIG. 5, power supply module 301 is illustrated according to an exemplary embodiment. In some embodiments, power supply module 301 houses power supply board 179. Power supply module 301 may perform and/or support the functions of power supply board 179 discussed with reference to FIG. 3 herein. These functions may include converting alternating current (e.g., from a mains power source) to direct current for use by components of portable ultrasound system 100, voltage transformations, and/or other power supply functions. Power supply module 301 may also provide short circuit protection, overpower protection, overvoltage protections, undervoltage protection, overcurrent protection, over temperature protection, and/or other types of electrical protection to portable ultrasound system 100 and the components thereof. Power supply module 301 alone or in conjunction with main circuit board module 401 and/or ultrasound module 201 may switch between power sources (e.g., a main battery, backup battery, and mains power). For example, mains power may be used when available to power imaging functions carried out by ultrasound module 201. When mains power is unavailable, power may be drawn from a battery power source. When the battery power source is depleted, a backup battery may provide an orderly shutdown of portable ultrasound system 100. Power supply module 301 alone or in conjunction with main circuit board module 401 and/or ultrasound module 201 may switch between power sources and manage power functions such as an orderly shutdown of portable ultrasound system 100.

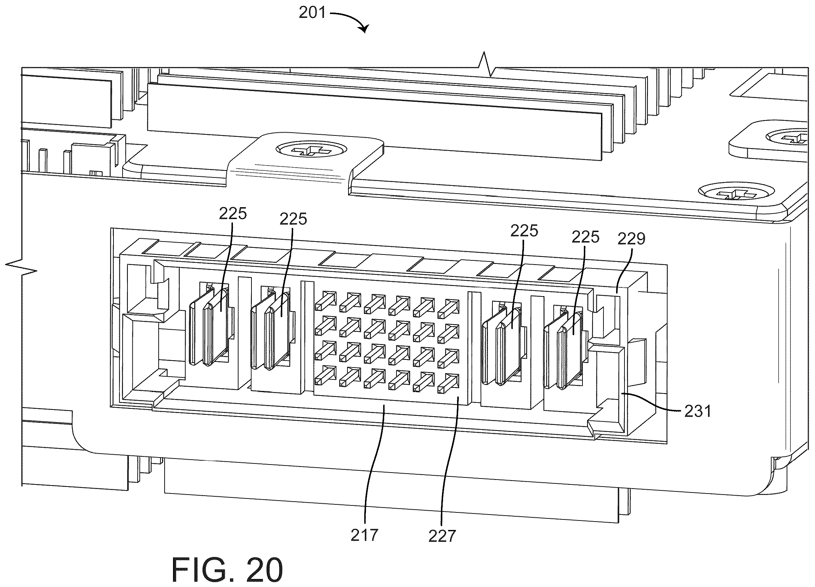

**In** some embodiments, power supply module 301 includes components which facilitate the modularity of portable ultrasound system 100. In one embodiment, power supply module 301 includes a floating connector 303. Floating connector 303 allows ultrasound module 201 to connect to power supply module 301. This connection may allow for power and/or data transfer between power supply module 301 and ultrasound module 201. Floating connector 303 is configured to move three dimensionally relative to power supply module 301 while still being electrically connected to the components of power supply module 301. This is described in more detail with reference to FIGS. 20-21.

Advantageously, the movement of floating connector 303 helps to align and connect ultrasound module 201 to power supply module 301 and thus allows for easier insertion of ultrasound module 201 into portable ultrasound system 100. Floating connector 303 may allow for connection between ultrasound module 201 and power supply module 301 to occur within a greater range of alignment tolerances as floating connector 303 is allowed to move relative to power supply module 301. Thus, less precision in the alignment of ultrasound module 201 and power supply module 301 may be required when inserting an ultrasound module 201 into portable ultrasound system 100. This may make it easier for a user to inset ultrasound modules 201 (e.g., with less attention to accuracy, greater speed, etc.). Advantageously, floating connector 303 may also reduce the stress experienced by connectors on the ultrasound module 201 by helping to guide the connectors into place with floating connector 303 while moving relative to power supply module 301. The floating nature of floating connector 303 may increase the alignment tolerances of the two sets of connectors (e.g., on ultrasound module 201 and power supply module 301) easing the connection process and helping to reduce stress. Advantageously, the reduced stress experienced by the connectors due to floating connector 303 may increase the life cycle of connectors on one or more of the ultrasound module 201 and the power supply module 301. This may reduce the amount of repair and/or maintenance performed on ultrasound module 201 and/or power supply module 301 and increase the amount of uptime for portable ultrasound system 100.

**In** some embodiments, power supply module 301 includes additional connectors and/or connections. For example, power supply module 301 may be connected to both ultrasound module 201 and main circuit board module 401. Power supply module 301 may be connected to ultrasound module 201 with floating connector 303. In some embodiments, power supply module 301 is also connected to main circuit board module 401 with a second floating connector 303. In other embodiments, power supply module 301 is connected to main circuit board module 401 with a fixed connector. In still further embodiments, power supply module 301 may be connected to main circuit board 401 with fixed connections and/or wiring. For example, power supply module 301 and main circuit board 401 may be configured so as to not be separable during normal operation. During repair and/or replacement of components, power supply module 301 and main circuit board 401 may be separated. For example, connectors and/or wiring could be de-soldered from one or both of the modules.

**In** some embodiments, power supply module 301 may be configured so as to be a replaceable and/or upgradable module. Power supply module 301 may be a contained module containing power supply components. This may allow for the removal of a power supply module 301 and for power supply module 301 to be replaced. Advantageously, the contained nature of power supply module 301 may allow for easy replacement of a power supply module 301 in order to repair or upgrade the power supply components of portable ultrasound system 100. This may allow for quick repairs to be made and for upgrades to be made in order to support other components of portable ultrasound system 100. For example, if an ultrasound module 201 is replaced with an ultrasound module 201 requiring different power specifications (e.g., a different operating voltage), then power supply module 301 may also be replaced to support the new ultrasound module 201. The modular nature of power supply module 301 may facilitate the repair or replacement of power components of portable ultrasound system 100. In some embodiments, power supply module 301 may be removed from portable ultrasound system 100 using a release lever. In other embodiments, power supply module 301 is attached to portable ultrasound system 100 (e.g., a frame and/or cover of the system) using screws, nuts and bolts, adhesive, and/or other fasteners.

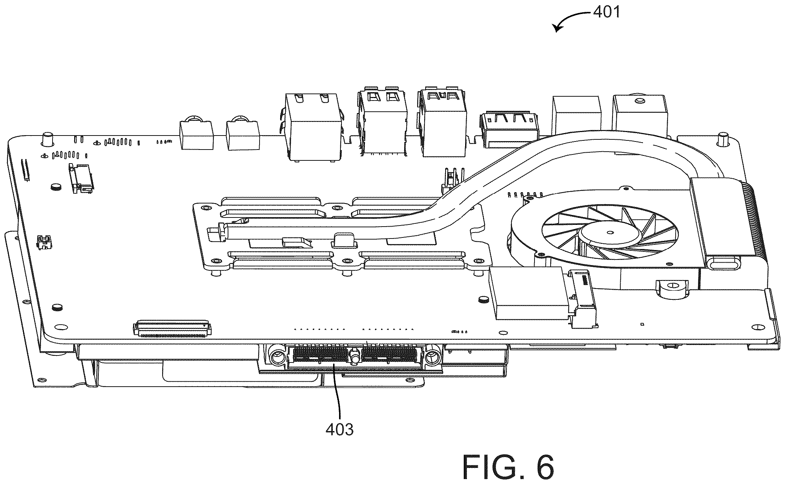

Referring now to FIG. 6, main circuit board module 401 is illustrated according to an exemplary embodiment. In some embodiments, main circuit board module 401 houses main circuit board 161. Main circuit board module 401 may perform and/or support the functions of main circuit board 161 discussed with reference to FIG. 3 herein. These functions may include performing general computations such as running an operating system for portable ultrasound system 100, handling inputs and generating outputs, displaying images, running applications, managing resources and/or other tasks performed by general computing hardware. Main circuit board module 401 may also perform communication functions. For example, main circuit board module 401 may send or receive data using a wireless communication device such as a radio transceiver. Main circuit board module 401 may also control components of portable ultrasound system 100. For example, main circuit board module 401 may use power supply module 301 to switch between power sources in some embodiments.

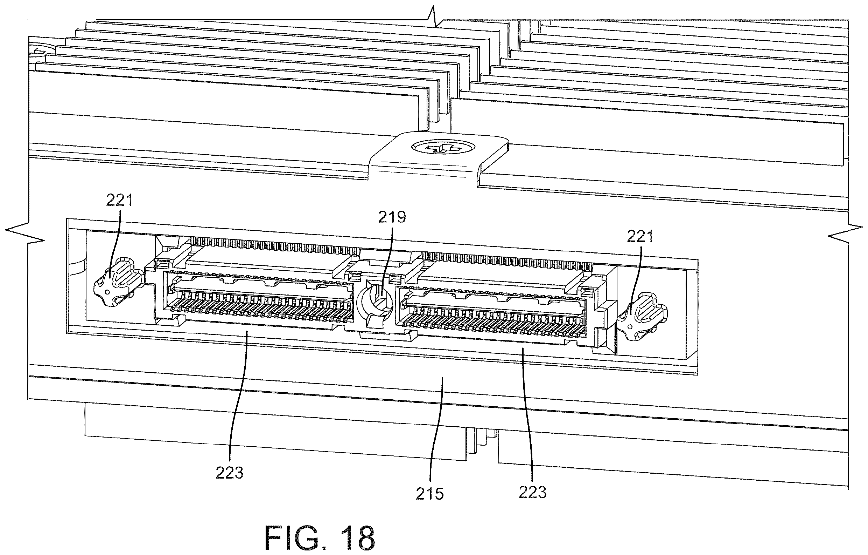

**In** some embodiments, main circuit board module 401 includes components which facilitate the modularity of portable ultrasound system 100. In one embodiment, main circuit board module 401 includes a fixed connector 403. Fixed connector 403 may allow main circuit board module 401 to connect to ultrasound module 201. This connection may allow for power and/or data transfer between main circuit board module 401 and ultrasound module 201. For example, main circuit board module 401 may send control instructions received through user input devices of portable ultrasound system 100 to ultrasound module 201. Continuing the example, ultrasound module 201 may send image data (e.g., bitmaps, frame buffers, sensor data, or other information related to imaging with ultrasound) to main circuit board module 401 for further processing and/or display on displays of portable ultrasound system 100. Fixed connector 403 may include features which allow for ultrasound modules 201 to be easily connected to or removed from main circuit board module 401. This is described in more detail with reference to FIGS. 18-19.

**In** some embodiments, main circuit board module 401 includes additional connectors and/or connections. For example, main circuit board module 401 may be connected to both ultrasound module 201 and power supply module 301. Main circuit board module 401 may be connected to ultrasound module 201 with fixed connector 403. In some embodiments, main circuit board module 401 is also connected to power supply module 301 with a second fixed connector 403. In other embodiments, main circuit board module 401 is connected to power supply module 301 with a floating connector. In still further embodiments, main circuit board 401 may be connected to power supply module 301 with fixed connections and/or wiring. For example, main circuit board 401 and power supply module 301 may be configured so as to not be separable during normal operation. During repair and/or replacement of components, main circuit board 401 and power supply module 301 may be separated. For example, connectors and/or wiring could be de-soldered or unconnected from one or both of the modules.

**In** some embodiments, main circuit board module 401 is configured so as to be a replaceable and/or upgradable module. Main circuit board module 401 may be a contained module containing computing components (e.g., such as the components discussed above with reference to main circuit board 161 and FIG. 3). This may allow for the removal of a main circuit board module 401 and for main circuit board module 401 to be replaced. Advantageously, the contained nature of main circuit board module 401 may allow for easy replacement of a main circuit board module 401 in order to repair or upgrade the computing components of portable ultrasound system 100. This may allow for quick repairs to be made and for upgrades to be made in order to support other components of portable ultrasound system 100. In some embodiments, main circuit board module 401 may be removed from portable ultrasound system 100 using a release lever. In other embodiments, main circuit board module 401 is attached to portable ultrasound system 100 (e.g., a frame and/or cover of the system) using screws, nuts and bolts, adhesive, and/or other fasteners.

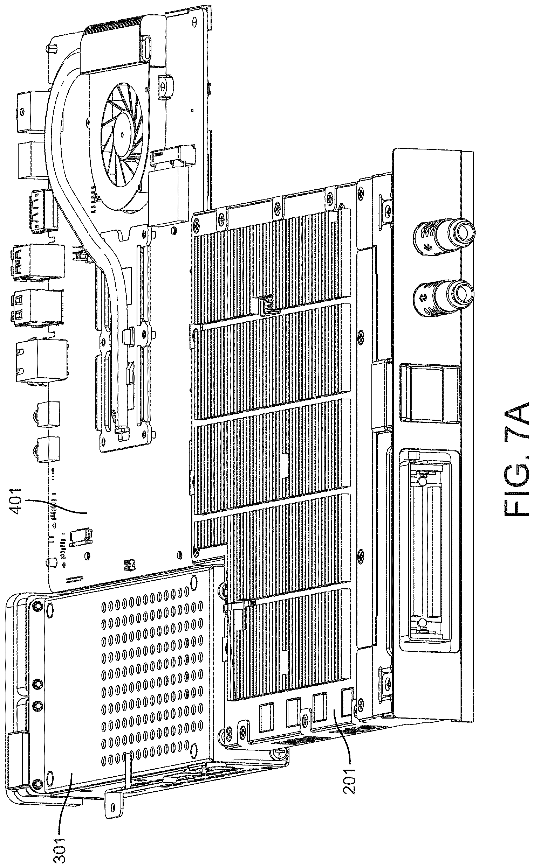

FIG. 7A illustrates an ultrasound module 201 connected to main circuit board module 401 and power supply module 301 according to an exemplary embodiment. In some embodiments, power and/or information is transferred between two or more of ultrasound module 201, power supply module 301, and main circuit board module 401. In one embodiment, ultrasound module 201, power supply module 301, and main circuit board module 401 are configured such that a portable ultrasound module 201 may be removed and/or connected to power supply module 301 and main circuit board module 401 simultaneously. For example, power supply module 301 and main circuit board module 401 may be arranged such that their respective connectors are parallel and aligned with corresponding connectors on an ultrasound module 201. As depicted in FIG. 7A, ultrasound module 201 may connect simultaneously to power supply module 301 and main circuit board module 401 through connectors on the rear of ultrasound module 201.

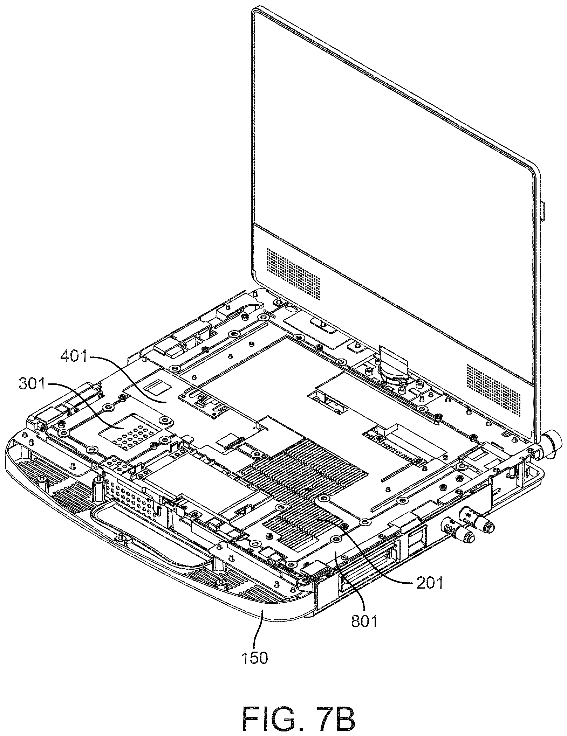

Now referring to FIG. 7B, ultrasound module 201, power supply module 301, and main circuit board module 401 may be supported by a single frame 801. In some embodiments, power supply module 301 and/or main circuit board module 401 are attached to frame 801. For example, power supply module 301 and/or main circuit board module 401 may be attached to frame 801 with screws, nuts and bolts, adhesive, and/or other fasteners. Frame 801 may position the connectors on power supply module 301 and main circuit board module 401 such that an ultrasound module 201 may simultaneously connect or disconnect from both power supply module 301 and main circuit board module 401. Frame 801 may also hold power supply module 301 and main circuit board module 401 in place while ultrasound module 201 is either connected or disconnected.

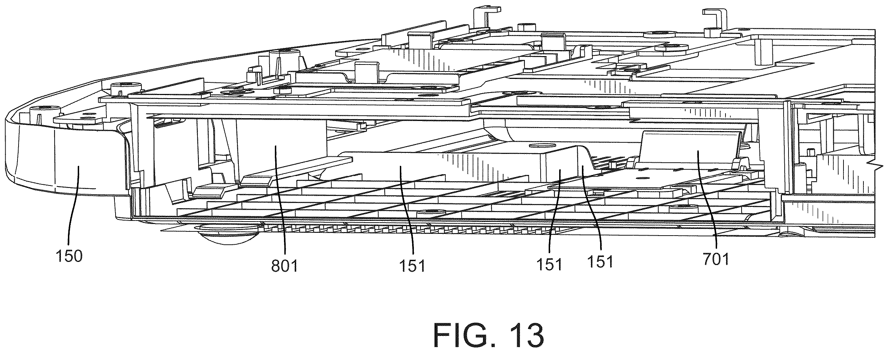

Other components of portable ultrasound system 100 may also be attached to frame 801. In some embodiments main housing 150 connects to frame 801. Other components such as the display (e.g., display swivel mechanism) may be attached to frame 801. In some embodiments, frame 801 secures ultrasound module 201 while it is inserted in portable ultrasound system 100. This is discussed in greater detail with reference to FIGS. 13-16.

Referring now to FIG. 8, an exemplary embodiment of portable ultrasound system 100 is illustrated showing an opening 803 to receive an ultrasound module 201. In some embodiments, opening 803 is defined by frame 801 and/or main housing 150. Opening 803 may be configured to allow an ultrasound module 201 to be inserted into portable ultrasound system 100. In one embodiment, opening 803 is located on a side of portable ultrasound system 100. For example, opening 803 may be located on the right side of portable ultrasound system 100. In other embodiments, opening 803 is located on a different side of portable ultrasound system 100. For example, opening 803 may be located on the left face of main housing 150, the front face of main housing 150, the rear face of main housing 150, or the bottom face of main housing 150. In some embodiments, the geometry of ultrasound module 201, power supply module 301, and/or the geometry of main circuit board housing 401 may be configured to fit within main housing 150 and work as described herein with respect to an opening 803 located on one of the faces of main housing 150.

**In** some embodiments, opening 803 is configured to prevent contaminates and/or particles from entering opening 803 when an ultrasound module 201 has been inserted. For example, opening 803 may be sized such that ultrasound module 201 overhangs opening 803 when inserted into portable ultrasound system 100. In other embodiments, opening 803 may include features such as a sealing material, gasket, door, flap, or other feature configured to prevent containments and/or particles from entering opening 803 when an ultrasound module 201 is inserted.

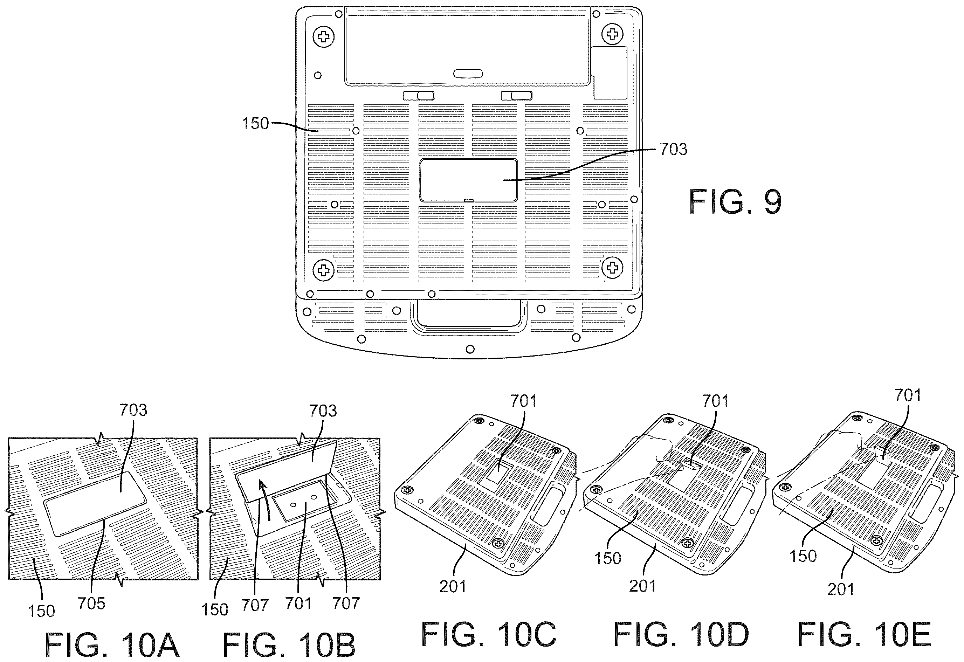

With reference to FIGS. 9-16, portable ultrasound system 100 may include features which support the modularity of the system by assisting in the insertion and removal of an ultrasound module 201. In some embodiments, portable ultrasound system 100 includes release lever 701. Advantageously, release lever 701 may assist a user in removing ultrasound module 201 by disconnecting ultrasound module 201 from other components of portable ultrasound system 100. Release lever 701 may be configured to apply a force perpendicular to a face of ultrasound module 201 and parallel to the axis on which ultrasound module 201 is connected to other components of portable ultrasound system 100. This may reduce the sheering force experienced by the connectors during removal of an ultrasound module 201. The connectors may experience a lower amount of sheering force when disconnected using release lever 701 than would be experienced if ultrasound module 201 was pulled out of portable ultrasound system 100. This may advantageously reduce the chance of damaging a connector during removal of an ultrasound module 201 and/or increase the life of an ultrasound module and/or power supply module 301 and main circuit board module 401.

In some embodiments, frame 801 may include elements or features which support the modular nature of portable ultrasound system 100. For example, frame 801 may include features which align or otherwise position ultrasound module 201 for connection to other components such as power supply module 301 and/or main circuit board module 401. Advantageously, frame 801 may assist a user in more accurately positioning ultrasound module 201 relative to power supply module 301 and/or main circuit board module 401 and thus ease the connection between these components when inserting ultrasound module 201 into portable ultrasound system 100. Frame 801 may also include elements or features which assist in securely connecting ultrasound module 201 to power supply module 301 and/or main circuit board module 401. Advantageously, this may prevent unintended disconnection of ultrasound module 201.

Referring now to FIG. 9, an exemplary embodiment of portable ultrasound system 100 is illustrated including release lever access door 703. In some embodiments, release lever access door 703 is located on the bottom of main housing 150. In other embodiments, release lever access door 703 is located on other faces of main housing 150. Release lever access door 703 covers release lever 701. Advantageously, this prevents unintentional actuation of release lever 701 which may cause disconnection of ultrasound module 201. As unintentional disconnection of ultrasound module 201 may disrupt imaging with portable ultrasound system 100 and/or cause other ill effects (e.g., loss of data) release lever access door 703 prevents unintentional disconnection of ultrasound module 201.

Referring now to FIGS. 10A-10E, the process of disconnecting an ultrasound module 201 from portable ultrasound system 100 is illustrated according to an exemplary embodiment. As illustrated in FIG. 10A, release lever access door 703 may be normally closed while an ultrasound module 201 is connected to portable ultrasound system 100. In some embodiments, release lever access door 703 includes a cutout 705. Cutout 705 may allow a user to grab, apply leverage, or otherwise open release lever access door 703. Cutout 705 may define an opening with main housing 150 which allows a user to apply force to the underside of release lever access door 703.

FIG. 10B illustrates an exemplary embodiment of portable ultrasound system 100 with release lever access door 703 open. Opening release lever access door 703, reveals release lever 701. In some embodiments, release lever access door 703 rotates about an axis defined by hinge 707 and/or an axel. The axle may provide an axial link between main housing 150 and release lever access door 703. The axle may define an axis about which release lever access door 703 rotates between an open position and a closed position. In some embodiments, the axle may be a rod or bar offset from an upper edge of main housing 150. The axle may extend longitudinally between a first end and a second end, each of which may be attached to main housing 150. In other embodiments, the axle may be a hinge, pivot joint, or other type of bearing providing a rotatable linkage between main housing 150 and release lever access door 703.

Hinges 707 are shown extending from an edge of main housing 150. Hinges 707 may be used to couple release lever access door 703 (e.g., releasably or permanently) to an axel and/or to main housing 150. The coupling between hinges 707 and an axle may define an axis about which release lever access door 703 may rotate between an open position and a closed position. In some embodiments, hinges 707 may be configured to allow for release lever access door 703 to be removed or decoupled from main housing 150 when in the open position. For example, hinges 707 may only partially enclose an axel of main housing 150 and when rotated to the open position provide an opening which allows release lever access door 703 to be removed (e.g., allow the axel to pass through the opening in hinges 707). In other embodiments, release lever access door 703 is configured such that release lever access door 703 is not removable from main housing 150. For example, hinges 707 may enclose an axel of main housing 150 such that the axel may not pass through hinges 707.

FIG. 10C illustrates an exemplary embodiment of portable ultrasound system 100 with release lever access door 703 removed. When opened and removed, release lever access door 703 reveals release lever 701. With release lever access door 703 removed and release lever 701 visible but not actuated, ultrasound module 201 remains inserted in portable ultrasound system 100. Ultrasound module 201 also remains connected to other systems (e.g., power supply module 301 and/or main circuit board module 401).

Referring now to FIG. 10D, a user may actuate release lever 701 by rotating release lever 701 away from main housing 150. In some embodiments, release lever 701 may extend above main housing 150 such that a user can grab, apply leverage, or otherwise actuate release lever 701. Release lever 701 may be positioned relative to main housing 150 such that a user may apply force to the underside of release lever 701. In some embodiments, a portion of release lever 701 extending inside main housing 150 applies force to ultrasound module 201 as release lever 701 is actuated by a user. This force may cause ultrasound module 201 to disconnect from other components of portable ultrasound system 100 and/or be ejected from portable ultrasound system 100. In some embodiments, release lever 701 applies force to ultrasound module 201 throughout its full range of motion. For example, if lease lever 701 has a range of motion between zero degrees and ninety degrees relative to main housing 150, force may be applied during rotation of release lever 701 from any angle greater than zero degrees to the maximum rotation of ninety degrees.

In other embodiments, release lever 701 only applies force to ultrasound module 201 during a portion of full range of motion of release lever 701. Release lever 701 may have one or more ranges during which no force is applied to ultrasound module 201 while release lever 701 is rotated through the ranges. For example, release lever 701 may rotate through zero degrees to twenty degrees without an internal (e.g., within main housing 150) portion of release lever 701 contacting or otherwise applying force to ultrasound module 201. Advantageously, this may prevent unintended removal of ultrasound module 201. A user may have to rotate release lever 701 beyond a certain threshold angle in order to start applying force to ultrasound module 201 and thereby ensure that the user intends to disconnect ultrasound module 201. In some embodiments, release lever 701 may be configured such that no force is applied to ultrasound module 201 as release lever 701 is rotated from a partially actuated to a fully actuated position. For example, release lever 701 may be configured such that the portion of release lever 701 internal to main housing 150 does not contact or otherwise apply force to ultrasound module 201 as release lever 701 is rotated between seventy and ninety degrees relative to main housing 150. Advantageously, this may ensure that ultrasound module 201 is still held in place and prevents ultrasound module 201 from unintentionally slipping or falling out of portable ultrasound system 100. Release lever 701 may be configured to prevent ultrasound module 201 from falling out of portable ultrasound system 100 as described above while still ensuring that enough force is applied to ultrasound module 201 for a sufficient range of rotation to allow a user to grab a partially extended ultrasound module 201.

Referring now to FIG. 10E, a partially extended ultrasound module 201 is shown according to an exemplary embodiment. After release lever 701 has been actuated, ultrasound module 201 is disconnected from other components of portable ultrasound system 100 and ultrasound module 201 extends from portable ultrasound system 100 such that a user may grab ultrasound module 201 and remove it. In some embodiments, ultrasound module 201 is disconnected from other components of portable ultrasound system 100 and/or is partially extended only after release lever 701 is fully actuated. In other embodiments, ultrasound module 201 may be disconnected from other components of portable ultrasound system 100 and/or partially extended from main housing 150 without release lever 701 being fully actuated. In one embodiment, release lever 701 has a range of motion between zero and ninety degrees of rotation relative to main housing 150. In other embodiments, the range of motion of release lever 701 is greater or lesser relative to main housing 150. For example, release lever 701 may have a range of motion of zero to 140 degrees relative to main housing 150. Continuing the example, release lever 701 may have a range of motion of zero to sixty degrees relative to main housing 150. In some embodiments, fully actuating release lever 701 disconnects ultrasound module 201 and extends ultrasound module approximately 2.5 centimeters away from opening 803 and/or main housing 150. In other embodiments, fully actuating release lever 701 causes ultrasound module 201 to be extended a greater or lesser amount.

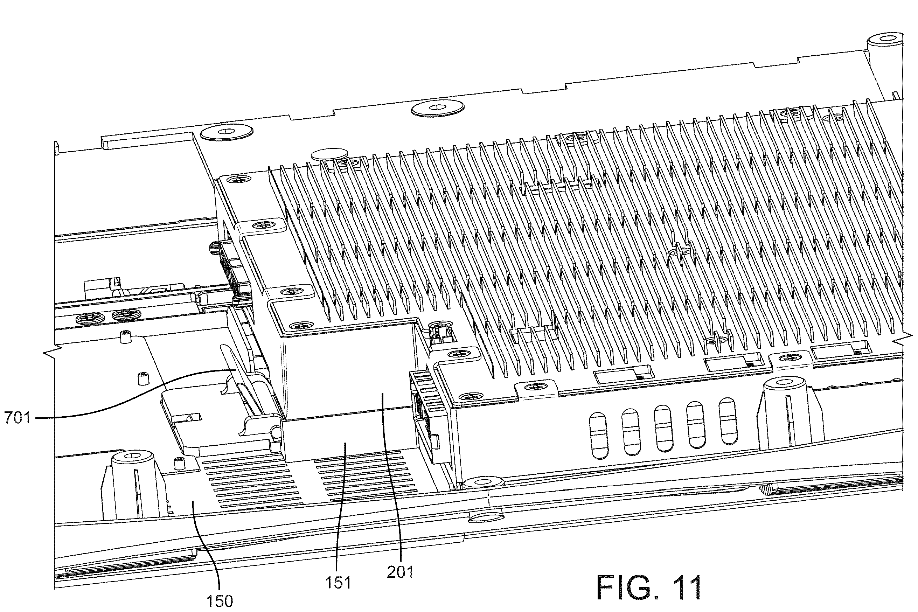

FIG. 11 illustrates a portion of release lever 701 extending within main housing 150 according to an exemplary embodiment. Release lever 701 may extend within main housing 150 such that rotation of a portion of release lever 701 accessible to the user (e.g., exterior to main housing 150) causes a corresponding rotation of a portion of release lever 701 internal to main housing 150. In some embodiments, release lever 701 is a single component extending both internally and externally to main housing 150. In other embodiments, release lever 701 may include multiple components configured to function as described herein.

Positioning of the lower portion (e.g., portion within main housing 150) relative to the upper portion (e.g., portion extending from main housing 150 and/or covered by release lever access door 703) may determine at what angle of rotation of release lever 701 force is applied to ultrasound module 201. For example, if the lower portion of release lever 701 is configured to be in contact with ultrasound module 201 while release lever 701 has rotated zero degrees relative to main housing 150, then any rotation of release lever 701 will begin to apply force to ultrasound module 201. The lower portion of release lever 701 may alternatively be positioned such that it is not in contact with an inserted ultrasound module 201 while release lever 701 has not been rotated. Depending on the angle formed by the lower portion of release lever 701 relative to the upper portion, the angle of rotation at which release lever 701 begins to apply force to ultrasound module 201 may be adjusted. By altering this angle, the above described functions of release lever 701 may be achieved.

In some embodiments, main housing 150 may include one or more shoulders 151. Shoulder 151 may guide, locate, prevent over insertion, and/or otherwise position ultrasound module 201. As illustrated in FIG. 11, release lever 701 may be positioned relative to an inserted ultrasound module. The ultrasound module's position may in turn be defined by one or more shoulders 151. The positioning of release lever 701 relative to shoulders 151 may define the characteristics of release lever 701 described above (e.g., at what rotation angle does release lever 701 begin to apply disconnecting force to ultrasound module 201). For example, release lever 701 as depicted in FIG. 11 does not begin applying disconnecting force to ultrasound module 201 until it has rotated approximately thirty degrees due to a user input on the upper portion of release lever 701.

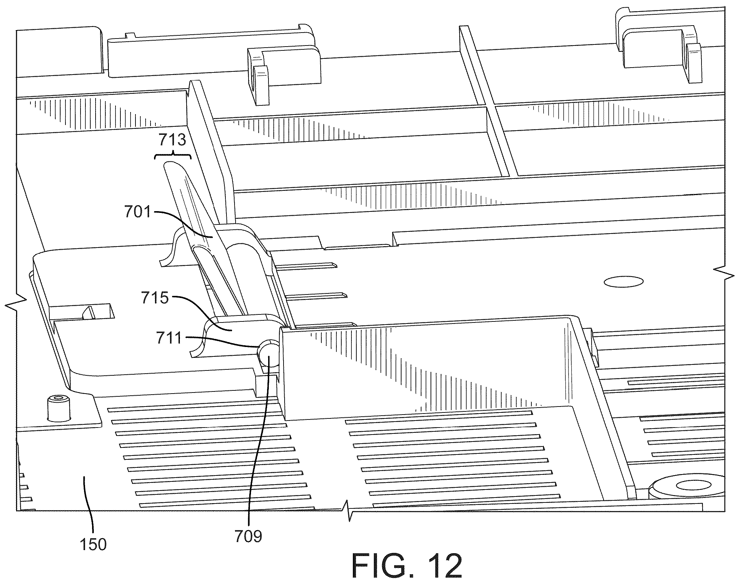

FIG. 12 illustrates one embodiment of release lever 701 and main housing 150 without an ultrasound module 201 inserted into portable ultrasound system 100. In one embodiment, release lever 701 is coupled to main housing 150 by axel 709 and opening 711. In some embodiments, axel 709 is coupled to flange 715 of main housing 150 such that axel 709 is held stationary. For example, axel 709 may be formed as part of main housing 150 through molding techniques, milled from main housing 150, inserted into opening 711 of flange 715 such that axel 709 has an interference fit with flange 715, coupled with an adhesive, or otherwise coupled to flange 715. Release lever 701 may include a sleeve which rotates about axel 709. Release lever 701 may rotate freely about axel 709. The upper and/or lower portions of release lever 701 may be attached to the sleeve. In some embodiments, the sleeve is an integral part of release lever 701. The sleeve and/or flange 715 may contain additional elements such as bearings.

In other embodiments, axel 709 is fixedly coupled to release lever 701. Axel 709 may be one or more protrusions from release lever 701. For example, axel 709 may be formed as an integral part of release lever 701 (e.g., by injection molding, milling, or another manufacturing technique). Axel 709 may be attached to release lever 701 using screws, nuts and bolts, adhesive, and/or other fasteners. Axel 709 may alternatively or in addition to the above techniques be inserted into a sleeve of release lever 701 such that the sleeve and axel 709 from an interference fit. Axel 709 may rotate freely within opening 711 of flange 715. For example, opening 711 and axel 709 may have a running fit. In some embodiments, opening 711 of flange 715, and/or axel 709 may be configured to support the rotation of axel 709 within opening 711. For example, a bearing assembly may be used to support axel 709 with flange 711.

In some embodiments, release lever 701 includes a cam portion 713. Cam portion 713 may function as a cam and facilitate the transformation of the rotational movement of release lever 701 into linear motion for pushing (e.g., disconnecting and ejecting) ultrasound module 201. The contact between cam portion 713 and ultrasound module 201 may be used to create linear motion of ultrasound module 201. Advantageously, this linear motion of ultrasound module 201 may reduce stress on the connectors of ultrasound module 201, power supply module 301, and/or main circuit board module 401 during disconnection of ultrasound module 201. In some embodiments, cam portion 713 has a cam profile to create uniform linear motion of ultrasound module 201 during the rotation of release lever 701 while disconnection ultrasound module 201. In additional embodiments, cam portion 713 has a cam profile which is configured to reduce stress and/or wear on release lever 701 and/or ultrasound module 201 due to the contact between release lever 701 and ultrasound module 201. Advantageously, this may increase the life span of release lever 701 and/or ultrasound module 201. Cam portion 713 may also provide an advantage to a user of portable ultrasound system 100 by creating uniform linear motion throughout the rotation of release lever 701. This may provide a user with predictable and repeatable disconnection of ultrasound module 201. In further embodiments, cam portion 713 may have a different cam profile. For example, cam portion 713 may have a cam profile which produces more linear motion for each degree of rotation of release lever 701. In other embodiments, cam portion 713 may have a cam profile which produces less linear motion per degree of release lever 701 rotation. This may allow for a release lever 701 with a greater range of rotation (e.g., 170 degrees).

In some embodiments, cam portion 713 extends for the entire length and/or width of the lower portion of release lever 701. In other embodiments, cam portion 713 is a portion of the lower portion of release lever 701.

In some embodiments, cam portion 713 has a cam profile which is configured to effect the range of motion of release lever 701 during which force is applied to ultrasound module 201. Cam portion 713 may have a profile which creates or helps to create the functions described above with reference to FIG. 11. For example, cam portion 713 may have a cam profile which results in no or little linear motion during a first range of rotation of release lever 701, linear motion during a second range of rotation of release lever 701, and no or little linear motion during a third range of rotation of release lever 701. Alternative profiles may be used to generate other functions described with reference to FIG. 11.

Advantageously, the configuration of cam portion 713 and/or the configuration of the upper and/or lower portions of the release lever 701 may provide a mechanical advantage to a user such that reduced force is required to disconnect an ultrasound module. This may make it easier for a user to disconnect and/or eject ultrasound module 201 from portable ultrasound system 100. This may also enhance the portability and modularity of portable ultrasound system 100 by making it easier to swap ultrasound modules 201 depending on the application and/or other needs of the user.

With reference to FIGS. 9-11, ultrasound module 201 may be disconnected from components of portable ultrasound system 100 using mechanisms other than release lever 701 as depicted. In some embodiments, release lever 701 is driven by electromechanical components rather than directly by a user. For example, the lower portion of release lever 701 may be driven by an actuator, solenoid, electric motor, or other electromechanical system. An electromechanical device may be used to drive a cam which provides linear motion to ultrasound module 201. In other embodiments, an electromechanical system may replace release lever 701. For example, an actuator, solenoid, motor and cam, or other electromechanical system may directly apply force to ultrasound module 201 to cause linear motion, disconnection, and/or ejection of ultrasound module 201.

In some embodiments, ultrasound module 201 is disconnected and/or ejected by the above described techniques in response to a user input received through a dedicated input mechanism. For example, the dedicated input mechanism may be a button, capacitive sensor, switch, knob, and/or other input device. The dedicated input mechanism may be accessed through a release lever access door 703 or like component. In other embodiments, the dedicated input mechanism is located elsewhere on portable ultrasound system 100. For example, the dedicated input mechanism may be located on a side of portable ultrasound system 100, included in a keyboard, located within main housing 150, be located adjacent to a display and/or input screen, be included in a cover or housing of main screen 130, or otherwise positioned in or on portable ultrasound system 100.

In other embodiments, ultrasound module 201 is disconnected and/or ejected by the above described techniques in response to a user input received through a user interface of portable ultrasound system 100. For example, the user interface of portable ultrasound system 100 may include an input element (e.g., a button, slider, radio button, field, or other graphical user interface element) which allows a user to disconnect and/or eject ultrasound module 201. Continuing the example, a user may provide an input using a button of the user interface and touchscreen 120 which causes portable ultrasound system 100 to activate an electromechanical system and thereby disconnect and/or eject ultrasound module 201.

In some embodiments, release lever 701 may include an interlock system. The interlock system may prevent disconnection and/or removal of ultrasound module 201. For example, the interlock system may prevent removal of ultrasound module 201 during inappropriate times (e.g., while imaging is in process, while data is being exchanged between ultrasound module 201 and main circuit board module 401, etc.) to prevent damage to components and/or loss of data. The interlock system may allow for removal of ultrasound module 201 when doing so will not cause harm. In one embodiment, the interlock system is controlled by main circuit board module 401. When main circuit board module 401 detects that it is not safe to disconnect ultrasound module 201 main circuit board module 401 may engage a normally open interlock mechanism. In other embodiments, the interlock mechanism, is normally closed (e.g., engaged to prevent removal of ultrasound module 201) and main circuit board module 401 disengages the interlock mechanism when it determines that it is safe to remove ultrasound module 201.

In some embodiments, the interlock mechanism may not be a physical mechanism but may be expressed in programming of portable ultrasound system 100. For example, a portable ultrasound system 100 may be programmed such that an electromechanical system which disconnects and/or ejects ultrasound module 201 may not be activated unless main circuit board module 401 determines that it is safe to disconnect and/or eject ultrasound module 201.

In other embodiments, the interlock mechanism is or includes physical components. For example, the interlock mechanism may be a lock or other physical component which impedes or prevents movement of release lever 701 when engaged. In further embodiments, the interlock mechanism may be a lock or other components which prevents a user from opening release lever access door 703 when engaged. In other embodiments, components other than main circuit board module 401 may perform the above described functions. For example, components of ultrasound module 201 (e.g., processors, memory, integrated circuits, etc.) may perform the tasks described above with reference to main circuit board module 401. In some embodiments, release lever access door 703 acts as an interlock mechanism.

Referring now to FIG. 13, release lever 701 is depicted attached to main housing 150 and with frame 801 also attached to main housing 150 according to an exemplary embodiment. In some embodiments, release lever 701 is attached to main housing 150 which is in turn attached to frame 801. In other embodiments, release lever 701 is attached to frame 801. Frame 801 and/or main housing 150 may position an inserted ultrasound module 201 relative to other components of portable ultrasound system 100. For example, features of main housing 150 and/or frame 801 may position ultrasound module 201 relative to release lever 701 such that release lever 701 functions as described above. Features of frame 801 and/or main housing 150 may position ultrasound module 201 such that ultrasound module 201 may be connected to power supply module 301 and/or main circuit board module 401.

In some embodiments, main housing 150 includes one or more shoulders 151. As previously discussed shoulder 151 may guide, locate, prevent over insertion, and/or otherwise position ultrasound module 201. Shoulders 151 may position ultrasound module 201 laterally and/or along the axis of insertion of ultrasound module 201. When ultrasound module 201 comes into contact with one or more shoulders 151, it is prevented from moving further in that direction. Advantageously, this may prevent excessive force, due to over insertion, on connectors of portable ultrasound system 100. This may also provide an advantage by aligning connectors on ultrasound module 201 with those on power supply module 301 and/or main circuit board module 401.

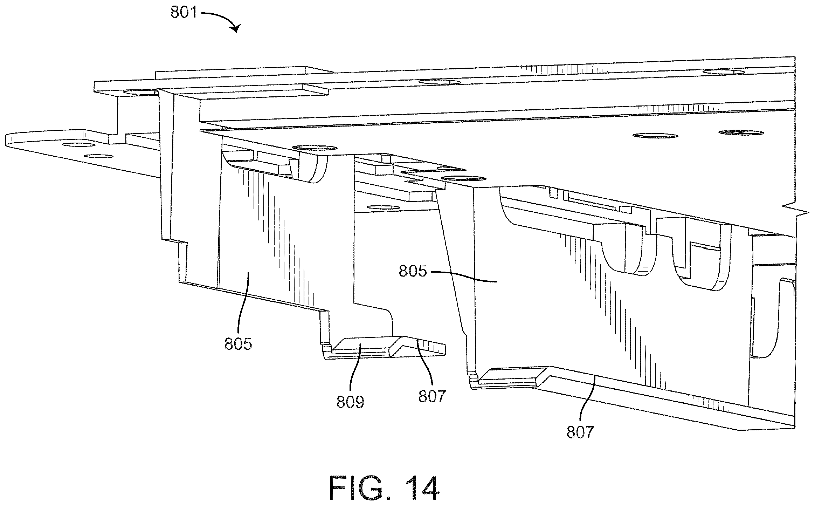

Referring now to FIG. 14, a section of frame 801 is illustrated according to an exemplary embodiment. In some embodiments, frame 801 includes one or more sides 805. Sides 805 may position ultrasound module 201 laterally within opening 803 and in relation to other components of portable ultrasound system 100. In some embodiments, frame 801 includes one or more floor portions 807. Floor portions 807 may position an ultrasound module vertically in relation to other components of portable ultrasound system 100. In some embodiments, floor portions 807 and/or sides 805 are positioned and/or configured such that ultrasound module 201 may be easily inserted and/or removed from portable ultrasound system 100. For example, floor portions 807 and/or sides 805 may be positioned and/or configured to create a free running fit with an inserted or partially inserted ultrasound module 201. In other embodiments, floor portions 807 and/or sides 805 are positioned and/or configured to create a fit with an inserted or partially inserted ultrasound module 201 such that ultrasound module 201 is held in place. For example, a close sliding fit may be used. Advantageously, this may hold ultrasound module 201 in place until sufficient force is applied by a user to remove ultrasound module 201 from portable ultrasound system 100, thereby preventing ultrasound module 201 from slipping out of the system. In additional embodiments, other fits may be formed by frame 801 and ultrasound module 201. In one embodiment, ultrasound module 201 is held in place by friction forces created by the connectors between ultrasound module 201 and power supply module 301 and/or main circuit board module 401. This is described in greater detail with reference to FIGS. 17-21.

In other embodiments, ultrasound module 201 is held in place by additional components rather than by a fit with frame 801. In one embodiment, frame 801 and/or ultrasound module 201 may include a friction material. The friction material may prevent ultrasound module 201 from sliding relative to frame 801 unless sufficient force is applied to overcome the static friction force created by the friction material. In other embodiments, ultrasound module 201 may be prevented from sliding due to plastically deformable protrusions included on frame 801 and/or ultrasound module 201. The protrusions may keep ultrasound module 201 from moving relative to frame 801 until a sufficient force is applied to plastically deform the protrusion. In additional embodiments, release lever 701 may include a component which latches to or otherwise connects to ultrasound module 201. Ultrasound module 201 may be releasably connected to release lever 701 which prevents movement of ultrasound module 201 relative to frame 801. When release lever 701 is actuated, release lever 701 may unlatch from ultrasound module 201. In some embodiments, a combination of the above described components and/or additional components may be used to prevent ultrasound module 201 from unintentionally sliding out of portable ultrasound system 100.

Still referring to FIG. 14, frame 801 may include one or more guides 809. Guides 809 may assist in inserting ultrasound module 201 into portable ultrasound system 100. Guides 809 may be a sloped segment of floor portions 807. Guides 809 may force an inserted ultrasound module 201 onto floor portion 807. Guides 809 may also allow an ultrasound module 201 to be inserted which is not already vertically aligned with floor portions 807. The slopping nature of guides 809 may cause insertion of ultrasound module 201 to align ultrasound module 201 vertically with floor portions 807. Advantageously, this may make it easier for a user to insert ultrasound module 201 as a user does not need to precisely align ultrasound module 201 with features of frame 801 and/or connectors of other components of portable ultrasound system 100. Guides 809 may more precisely align ultrasound module 201 with frame 801 and in turn more precisely align ultrasound module 201 with connectors associated with other components of portable ultrasound system 100. Frame 801 may both support and assist in aligning ultrasound module 201. In further embodiments, guides 809 are also included in sides 805 and assist in aligning ultrasound module 201 laterally.

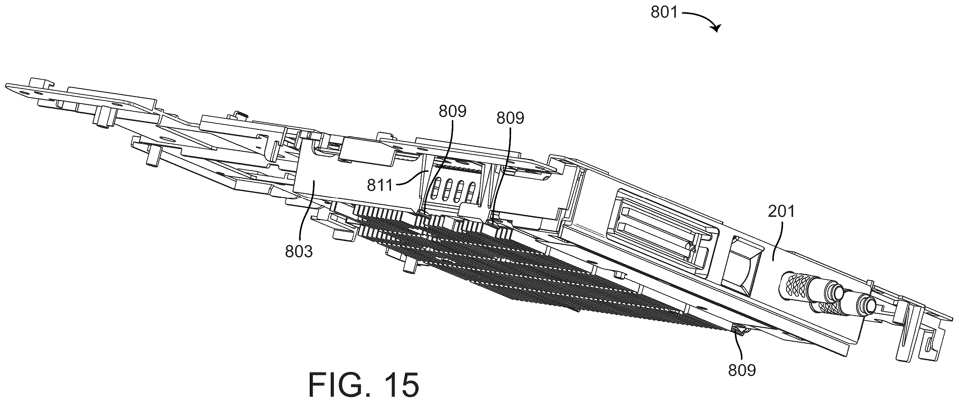

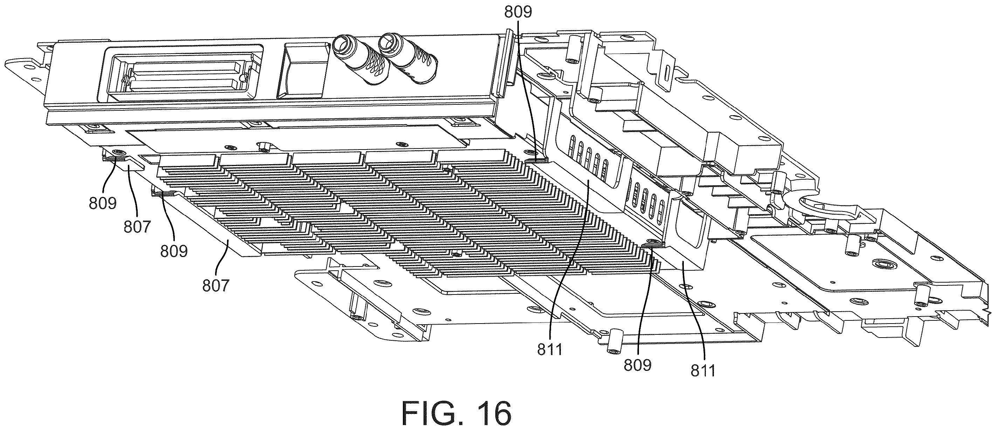

Referring now to FIG. 15, one embodiment of frame 801 is illustrated with ultrasound module 201 inserted. Sides 803 may align ultrasound module 201 laterally as described above. As illustrated, guides 809 extend below ultrasound module 201 when it is inserted in some embodiments. This allows ultrasound module 201 to be inserted without being vertically aligned with the surface of frame 801 on which is supported (e.g., floor portions 807). This is further illustrated in FIG. 16 according to an exemplary embodiment. Guides 809 may be located on left and right side of ultrasound module 201.

Referring now to FIG. 17, ultrasound module 201 includes two connectors according to some embodiments. Ultrasound module 201 may include female fixed connector 215. Female fixed connector 215 may be a connector which receives a male fixed connector on another component of portable ultrasound system 100. When female fixed connector 215 and a corresponding male fixed connector are in contact (e.g., when the male fixed connector is inserted into female fixed connector 215), an electrical connection may be exist between the two connectors. The electrical connection may allow for transmission of data and/or electrical power between the two components which are connected using the fixed connector pair.

In one embodiment, ultrasound module 201 includes female fixed connector 215 for connecting to main circuit board module 401. Main circuit board module 401 may include a male fixed connector for connecting to ultrasound module 201 via female fixed connector 215.

Ultrasound module 201 may also include male floating connector 217. In one embodiment, male floating connector 217 connects ultrasound module 201 to a female floating connector included in power supply module 301. In some embodiments, male floating connector 217 does not float (e.g., does not move relative to ultrasound module 201 to facilitate the connection of misaligned connectors), but is instead configured to connect to a female floating connector included in power supply module 301. The female floating connector may float as described herein with reference to FIGS. 20-21. In other embodiments, the male floating connector included in ultrasound module 201 floats and the female floating connector of power supply module 301 is fixed. In still further embodiments, both connectors of the floating connector pair float.

Advantageously, the features described herein with respect to FIGS. 17-21 may allow for ultrasound module 201 to easily connect with other components of portable ultrasound system 100 (e.g., power supply module 301 and/or main circuit board module 401). The connectors may be configured to aid in alignment of the modules and/or connector components such as the contacts which allow data and/or power transfer between modules via electrical communication through the contacts. The connectors may be configured to self-align or to otherwise tolerate some misalignment between ultrasound module 201 and power supply module 301 and/or main circuit board module 401. Advantageously this may make it easier for a user to insert and connect an ultrasound module 201 to portable ultrasound system 100. Furthermore, wear and/or stress on components of the connectors (e.g., the contacts) may be reduced by handling misalignment and therefore increase the life cycle of the connectors.

In some embodiments, ultrasound module 201 and/or other components of portable ultrasound system 100 may have different connections, a greater number of connections, a lesser number of connections, and/or other configurations than are described herein with reference to FIGS. 17-21. For example, ultrasound module 201 may have a male fixed connector 407 and/or a female floating connector 307 rather than the counterparts described above. In further embodiments, ultrasound module 201 may include additional and/or different connectors.

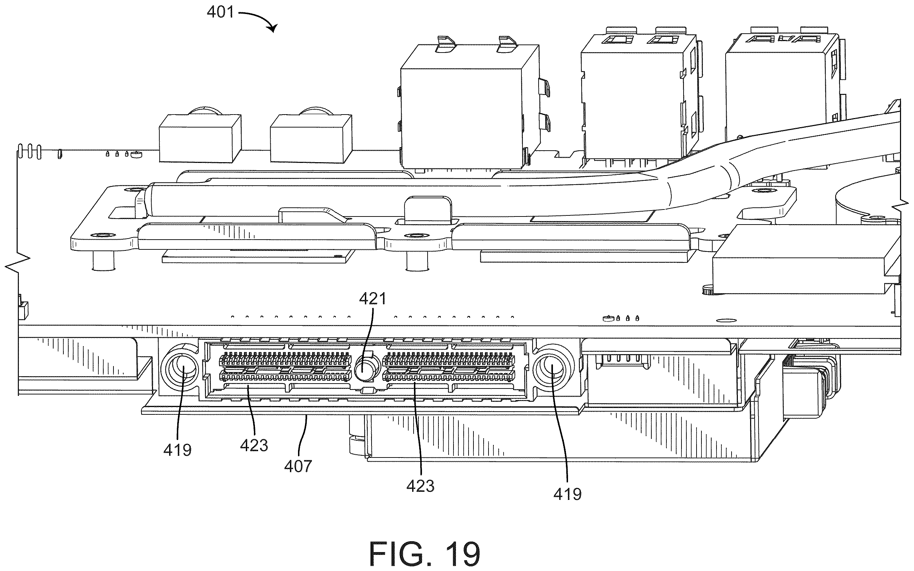

Referring now to FIGS. 18 and 19, a female fixed connector 215 of ultrasound module 201 and a male fixed connector 407 of main circuit board module 401 are illustrated according to an exemplary embodiment. The pair of fixed connectors (e.g., female fixed connector 215 and male fixed connector 407) may allow for ultrasound module 201 to be in communication with main circuit board module 401. This connection may allow for the transfer of data, instructions, control signals, information, electrical power, and/or other signals or information between ultrasound module 201 and main circuit board module 401. Communication between ultrasound module 201 and main circuit board module 401 may take place via an electrical connection formed between contacts 213 on female fixed connector 215 of ultrasound module 201 and contacts 423 of male fixed connector 407 of main circuit board module 401. When the connector pair is connected (e.g., male fixed connector 407 is inserted into female fixed connector 215), contacts 223 and contacts 423 may be in physical communication thereby allowing electrical communication (e.g., contacts 223 and contacts 423 are made of one or more conducting materials).

In some embodiments, the fixed connector pair include elements to facilitate connection between female fixed connector 215 and male fixed connector 407. These elements may align the two connectors and/or otherwise correct misalignment of the two connectors as ultrasound module 201 is inserted and/or connected to portable ultrasound system 100. In some embodiments, female fixed connector 215 includes one or more guide slots 219. Guide slots 219 may correspond to one or more guide shafts 421 of male fixed connector 407. Guide slot 219 may be configured to receive a guide shaft 421 such that guide slot 219 may receive guide shaft 421 when female fixed connector 215 and male fixed connector 407 are misaligned. For example, guide slot 219 may have an outer radius greater than the radius of guide shaft 421. As guide shaft 421 is inserted into guide slot 219, the female fixed connector 215 and male fixed connector 407 may be aligned. In some embodiments, the radius of guide slot 219 decreases along the depth of guide slot 219. As guide shaft 421 travels deeper into guide slot 219 the decreasing radius of guide slot 219 may center guide shaft 421 within guide slot 219. As guide shaft 421 is centered in guide slot 219, female fixed connector 215 and male fixed connector 407 may be aligned. Advantageously, this allows misaligned connectors to be aligned as ultrasound module 201 is inserted. In some embodiments, guide shaft 421 has a decreasing radius extending outward from male fixed connector 407. This may allow for easier alignment of guide shaft 421 and guide slot 219. Contacts 223 of female fixed connector 215 may be brought into alignment with contacts 423 of male fixed connected 407 by one or more of these features.

In some embodiments, female fixed connector 215 includes guide shafts 221 which correspond to guide slots 419 on male fixed connector 407. As explained above, guide shafts 221 and the corresponding guide slots 419 may align female fixed connector 215 and male fixed connector 407 as guide shafts 221 are inserted into guide slots 419. Guide shafts may be inserted into guide slots as ultrasound module 201 is inserted into portable ultrasound system 100.

In some embodiments, contacts 423 of male fixed connector 407 and contacts 223 of female fixed connector 215 are configured to tolerate some misalignment between male fixed connector 407 and female fixed connector 215. For example, contacts 223 of female fixed connector 215 may include chamfered features which align the contacts 423 of male fixed connector 407 as they are inserted. Contacts 423 of male fixed connector 407 may also be configured to align with contacts 223 of female fixed connector 215. For example, connectors 423 of male fixed connector 407 may be sized to fit within female fixed connector 215.

In some embodiments, male fixed connector 407 and/or female fixed connector 215 are configured to provide friction force to secure ultrasound module 201 within portable ultrasound system 100 when inserted. The friction force may prevent or assist in preventing ultrasound module 201 from disconnecting from the other components of portable ultrasound system 100 except by the function of release lever 701. For example, the guide shaft and guide slot features described above may be sized such that an inserted ultrasound module 201 is held in place by the friction between a guide shaft and the guide slot into which the guide shaft has been inserted. Once a guide shaft has been aligned by and inserted fully into a guide slot, the inner radius of the guide slot and the guide shaft may form an interference fit or other type of fit to provide friction force. The fit may still allow removal of the guide shaft from the guide slot in response to force provided by release lever 701.

Female fixed connector 215 and the associated features have been described as corresponding to ultrasound module 201 and male fixed connector 407 and the associated features have been described as corresponding to main circuit board module 401. However, this is illustrative only. In other embodiments, fixed connectors (male and/or female) may be used on other or additional components. For example, male fixed connector 407 may be located on ultrasound module 201 and female fixed connector 215 may be located on main circuit board module 401. In further embodiments, fixed connectors may be used to couple ultrasound module 201 and other components of portable ultrasound system 100 (e.g., power supply module 301).

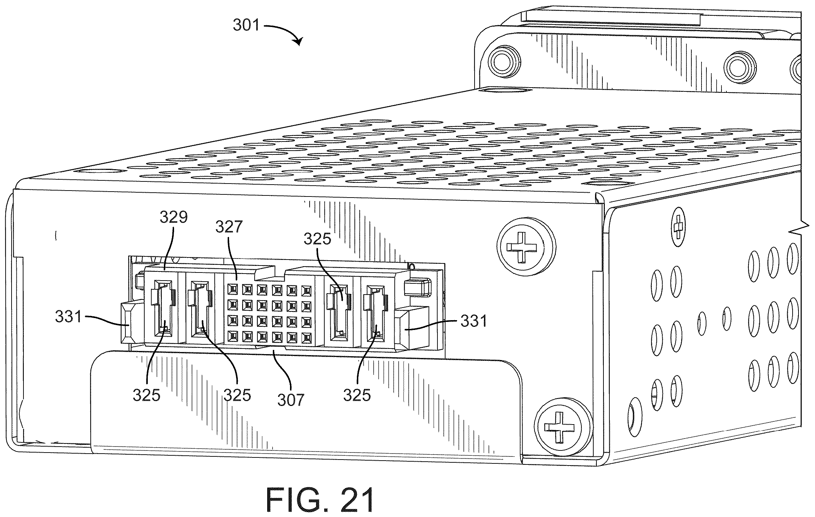

Referring now to FIGS. 20 and 21 a pair of floating connectors are illustrated according to an exemplary embodiment. In one embodiment, ultrasound module 201 includes one or more male floating connectors 217. Male floating connector 217 is configured to connect to female floating connector 307. In one embodiment, female floating connector 307 is coupled to power supply module 301. In some embodiments, male floating connector 217 on ultrasound module 201 is fixed while female floating connector 307 is floating as described below. In other embodiments, either floating connector or both floating connectors may be floating. In further embodiments, both floating connectors may be fixed.

In some embodiments, male floating connector 217 includes one or more types of contacts to establish electrical communication with the contacts of female floating connector 307. For example, male floating connector 217 may include pin contacts 227. Pin contacts 227 may be configured to come into electrical contact with pin slot contacts 327 of female floating connector 307 when male floating connector 217 is connected to female floating connector 307. Pin slot contacts 327 and pin contacts 227 may be made of a conductive material to allow electrical communication between both sets of contacts. In some embodiments, pin slot contacts 327 include chamfered openings to the slots which assist in the alignment of pin contacts 227 and pin slot contacts 327. The reducing openings of pin slot contacts 327 may allow pin contacts 227 to be inserted and then centered with pin slot contacts 327 as the size of the opening decreases along the depth of slot contacts 327.

Male floating connector 217 may include one or more plate contacts 225. In some embodiments, plate contacts 225 are configured to push outward so as to be in contact with plate slot contacts 325 of female floating connector 307 when male floating connector 217 is inserted. For example, plate contacts 225 may be spring loaded so as to push outward against plate slot contacts 325, positioned such that they plastically deform and push against plate slot contacts 325 when inserted, or otherwise configured to form an electrical connection with plate slot contacts 325. In some embodiments, plate slot contacts 325 are chamfered to aid in the alignment of plate contacts 225 with plate slot contacts 325. The electrical communication formed by contacts on male floating connector 217 and female floating connector 307 may allow for the transfer of electrical power, control signals, data, and/or other information between ultrasound module 201 and power supply module 301. In one embodiment, electrical power is provided from power supply module 301 to ultrasound module 201 using plate contacts 225 and plate slot contacts 325. In further embodiments, data, control signals, and/or other information is transferred between ultrasound module 201 and power supply module 301 using pin contacts 227 and pin slot contacts 327.

In some embodiments, the pair of floating connectors includes guiding features which assist in aligning female floating connector 307 with male floating connector 217. For example, female floating connector 307 may include one or more guide protrusions 331. Guide protrusions may extend from female floating connector 307 and be chamfered. During connection of female floating connector 307 and male floating connector 217, guide protrusions 331 may be received by guide sections 231 of male floating connector 217. Guide sections 231 may also be chamfered. As the size of the above features is reduced along the depth of the guide feature, guide protrusions 331 may be inserted into guide sections 231 while misaligned. As guide protrusions 331 are inserted along the depth of guide section 231 the reducing size (e.g., due to the chamfer) of guide section 231 aligns male floating connector 217 with female floating connector 307. In some embodiments, the edge of housing 229 of male floating connector 217 is also chamfered. This may allow the chamfered main body 329 of female floating connector 307 to align with housing 229 of male floating connector 217 as described with reference to guide protrusions 331 and guide section 231.

In some embodiments, male floating connector 217 and/or female floating connector 307 are configured to provide friction force to secure ultrasound module 201 within portable ultrasound system 100 when inserted. The friction force may prevent or assist in preventing ultrasound module 201 from disconnecting from the other components of portable ultrasound system 100 except by the function of release lever 701. For example, the guide protrusion, guide section, housing, and/or main body features described above may be sized such that an inserted ultrasound module 201 is held in place by the friction between the features on male floating connection 217 and the features on female floating connector 307. For example, the fit between guide protrusion 331 and guide section 231 may form an interference fit or other type of fit to provide friction force. The fit may still allow removal of the guide shaft from the guide slot in response to force provided by release lever 701.

In further embodiments, female floating connector 307 floats in relation to its corresponding component (e.g., power supply module 301). Female floating connector 307 may have six degrees of freedom in relation to power supply module 301. This may allow female floating connector 307 to translate vertically, horizontally, and/or along the depth of power supply module 301. Female floating connector 307 may also pitch, roll, and/or yaw in relation to power supply module 301. Advantageously, this movement may allow female floating connector 307 to align itself with a male floating connector 217 that is initially misaligned. The floating nature of female floating connector 307 may augment the alignment features previously discussed such that a connection may be made between connectors which are misaligned to a greater degree. In some embodiments, female floating connector 307 is constrained in its six degrees of freedom by the features which allow it to float and/or the features connecting it to power supply module 301.

In some embodiments, female floating connector 307 is attached to power supply module 301 with one or springs which provide female floating connector 307 with six degrees of freedom. For example, a spring at each corner of female floating connector 307 may connect female floating connector 307 to power supply module 301. In some embodiments, the springs may run within guiding features which constrain the movement of female floating connector 307. For example, each spring may be positioned within a hollow shaft which runs within a slot connected to power supply module 301. The slot may be sized larger than the shaft to allow the shaft to move relative to the slot thereby giving female floating connector 307 six degrees of freedom. The springs may cause female floating connector 307 to return to an initial position and/or provide a force which assists in the alignment process (e.g., by brining female floating connector 307 into alignment with male floating connector 217 as the two connectors are aligned and/or connected). In other embodiments, plastically deformable features connect female floating connector 307 with power supply module 301. For example, female floating connector 307 may be attached to power supply module 301 by a flexible gasket. The gasket may position female floating connector 307 relative to the housing of power supply module 301 (e.g., the gasket may be positioned around female floating connector 307 and between female floating connector 307 and an opening in the housing of power supply module 301). Other techniques may be used to give female floating connector 307 six or fewer degrees of freedom relative power supply module 301.

In some embodiments, female floating connector 307 may include flexible features which connect pin slot contacts 327 and/or plate slot contacts 325 to fixed components of power supply module 301. Advantageously, this may allow for female floating connector 307 to move relative power supply module 301 without causing damage to the contacts or disconnecting the contacts from other components of power supply module 301. For example, pin slot contacts 327 and/or plate slot contacts 325 may be connected to other components of power supply module 301 with flexible wire long enough to allow movement of female floating connector 307 and the contacts therein. When connected, the contacts of female floating connector 307 and male floating connector 217 may allow fbr the communication of electrical power and/or data between ultrasound module 201 and power supply module 301. For example, data may be electrically communicated between ultrasound module 201 and power supply module 301 using the connection formed by pin contacts 227 and pin slot contacts 327. Continuing the example, electrical power may be electrically communicated between ultrasound module 201 and power supply module 301 using the connection formed by plate contacts 225 and plate slot contacts 325.

Female floating connector 307 and the associated features have been described as corresponding to power supply module 301 and male floating connector 217 and the associated features have been described as corresponding to ultrasound module 201. Additionally, female floating connector 307 has been described as floating and male floating connector 217 as fixed. However, this description is illustrative only. In other embodiments, floating connectors (male and/or female) may be used on other or additional components. For example, female floating connector 307 may be located on ultrasound module 201 and male floating connector 217 may be located on power supply module 301. In further embodiments, fixed connectors may be used to couple ultrasound module 201 and other components of portable ultrasound system 100 (e.g., power supply module 301). Additionally, both floating connectors float (e.g., have six degrees of freedom) in some embodiments. In other embodiments, male floating connector 217 floats and female floating connector 307 is fixed.

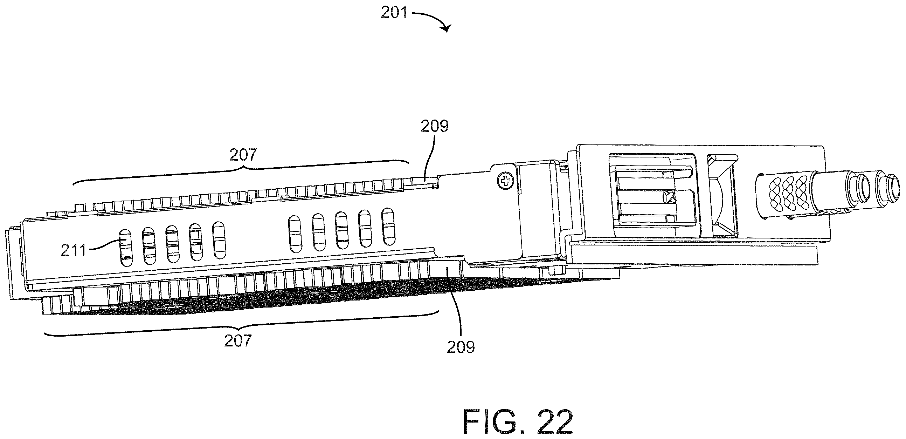

Referring now to FIGS. 22-26, portable ultrasound system 100 and/or ultrasound module 201 may include heat management features. Ultrasound components may give off heat which may increase the temperature of components within ultrasound module 201 and/or other components of portable ultrasound system 100. Components (e.g., processors, ASIC, memory, etc.) may perform better at lower temperatures. Additionally, a user may prefer that external components of portable ultrasound system 100 (e.g., input devices such as touchscreen 120, housing 150, or other components which a user may touch or come into contact with) remain cool to the touch. This may allow a user to more comfortably interact with portable ultrasound system 100. In turn, a user may be able to work longer with portable ultrasound system 100 because it is cool to the touch thereby increasing the efficiency of the user and the amount of work which may be done in a single sitting with portable ultrasound device 100. Advantageously, the heat management features of portable ultrasound system 100 may maintain internal and/or external components of portable ultrasound system 100 at a lower temperature. Thus, external components may be cool to a user's touch and internal components may function within appropriate temperature tolerances.

Referring now to FIG. 22, the heat management features of portable ultrasound system 100 may include heat sinks 207. In some embodiments, ultrasound module 201 two heat sinks 207. One heat sink 207 may be located on the upper surface of ultrasound module 201 with a second heat sink 207 located on the lower surface of ultrasound module 201. In other embodiments, other numbers of heat sinks may be used (e.g., one, three, four, etc.) and/or heat sinks 207 may be located in other locations on ultrasound module 201 (e.g., front, left side, right side, back, or other face of ultrasound module 201). In further embodiments, ultrasound module 201 may include cutouts 211. Cutouts 211 may create an air path internal to ultrasound module 201. The internal air path of ultrasound module 201 may allow for components within ultrasound module 201 to be cooled directly by convection (e.g., air is drawn through cutouts 211 via the air path using a fan). Combined with cooling from an air path above ultrasound module 201 and the associated heat sink 207 and combined with an air path below ultrasound module 201 and the associated heat sink 207, the internal air path may cool components of ultrasound module 201 and/or other components of portable ultrasound system 100.

In some embodiments, additional components of portable ultrasound system 100 may facilitate cooling of ultrasound module 201. For example, other components may create an air path for cooling ultrasound module 201. Referring now to FIGS. 15-16, frame 801 may include air path cutouts 811. Air path cutouts 811 in frame 801 may allow air to reach ultrasound module 201 when ultrasound module is connector to components of portable ultrasound system 100 and/or inserted into portable ultrasound system 100. This air may be used for cooling components of ultrasound module 201.

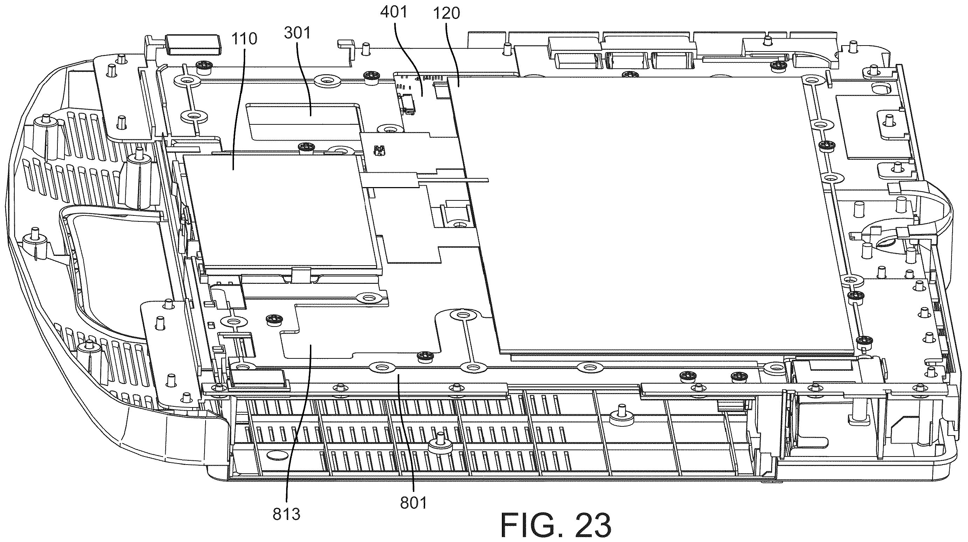

Referring now to FIGS. 23-26, the heat management features of portable ultrasound system 100 may include one or more heat shields 813 in some embodiments. In some embodiments, heat shield 813 is used to protect one component or a series of components in portable ultrasound system 100 from heat generated by other components or a series of components. In other embodiments, heat shields 813 may be used to protect a user from heat generated by components of portable ultrasound system 100. In further embodiments, heat shield 813 may be used to protect components of portable ultrasound system 100 from external sources of heat (e.g., ambient heat in an operating environment).

Heat shield 813 may be a substance, material or materials, and/or layer included in portable ultrasound system 100 which absorbs, dissipates, and/or reflects heat. Absorbed heat may be directed away from protected components by the heat shield 813. Heat shield 813 may operate on the principles of convection, conduction, reflection, absorption, or other thermodynamic or heat transfer principles for preventing heat from reaching a shielded component or components. In some embodiments, heat shield 813 includes or is an insulating material. Materials making up heat shield 813 may include heat conductive materials (e.g., copper, aluminum, etc.), heat reflective materials (e.g., foils, radiant barriers, metalized fabrics, laminate films, or other materials suitable for reflecting heat and/or radiation), insulating materials (e.g., ceramics, fiberglass, foams, polymers, fiber based materials, or other materials suitable for impeding heat transfer), backing materials (cloth, fabric, paper, metals, ceramics, of other materials suitable for providing mechanical support to the materials described herein), and/or other materials for shielding components from a source of heat. In some embodiments, heat shield 813 may include an air space (e.g., as a layer between layers of other materials) and/or be positioned relative to other components of portable ultrasound system 100 such that an air space is created between heat shield 813 and the component to be shielded and/or the heat source. In additional embodiments, heat shield 813 is or includes an ablative heat shield and/or a thermal soak heat shield.

With reference to FIG. 23, heat shield 813 may be positioned and/or otherwise configured to shield components of portable ultrasound system 100 from heat generated by ultrasound module 201. In one embodiment, heat shield 813 is a layer attached to frame 801 and positioned between touchscreen 120 and ultrasound module 201 and/or opening 803 for ultrasound module 201. For example, heat shield 813 may be a Mylar (e.g., biaxially-oriented polyethylene terephthalate) sheet, panel, rigid layer, flexible layer, and/or include additional materials such as heat reflecting materials, insulating materials, backing materials, or other materials related to heat management. In some embodiments, heat shield 813 is attached to frame 801 with one or more of screws, nuts and bolts, clips, adhesive, and/or other fasteners.

Advantageously, this positioning of heat shield 813 may shield one or more of touchscreen 120, touchpad 110, and a keyboard from heat generated by components of portable ultrasound system 100. This may protect these or other components from damage caused by exposure to heat and/or improve the user experience by providing user inputs which are not hot or warm to the touch. Particularly, heat shield 813 may provide an advantage by preventing heat generated by ultrasound module 201 from increasing the temperature of user input device touchscreen 201. This may provide a particular advantage as components of ultrasound module 201 may produce a larger amount of heat or a larger amount of heat than other components of portable ultrasound system 100. Additionally, heat shield 813 may allow for portable ultrasound system 100 to be packaged in such a way as to support the modularity of the system. For example, heat shield 813 may allow for ultrasound module 201 to be located below components such as touchscreen 120 without the heat from ultrasound module 201 creating adverse effects in other components. Heat shield 813 may also allow the overall size of portable ultrasound system 100 to be reduced (e.g., the system may be thinner by allowing ultrasound module 813 to be closer to touchscreen 120) thus increasing the portability of the system.

In other embodiments, heat shield 813 extends to shield touchpad 110 and/or a keyboard. This may provide an advantage of decreasing the temperature of these components felt by a user and/or increasing the performance or durability of these components. In further embodiments, a plurality of heat shields 813 are used to protect components of portable ultrasound system 100.

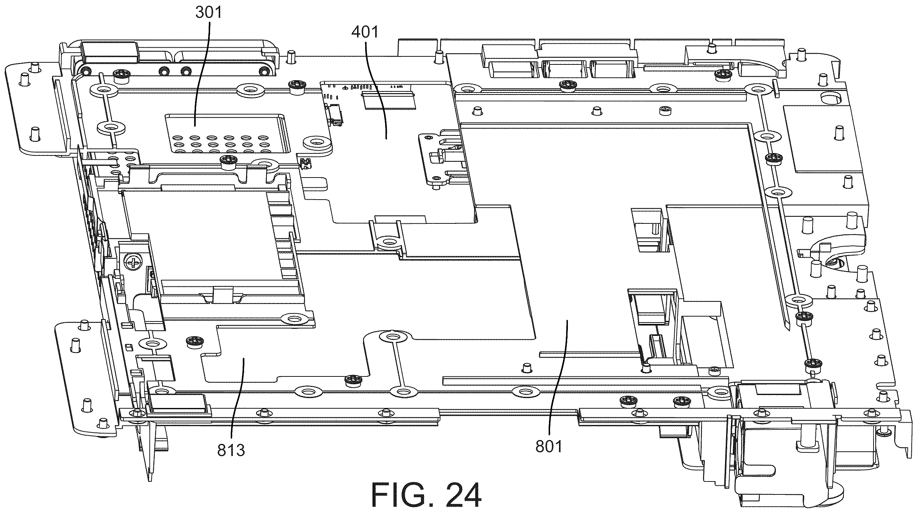

Referring now to FIG. 24, heat shield 813 may be attached to the underside of frame 801 in one embodiment. Touchscreen 120 may be positioned on top of frame 801. In some embodiments, frame 801 is configured to shield touchscreen 120 from heat in conjunction with heat shield 813. For example, frame 801 may include a solid portion beneath touchscreen 120 which acts as an insulating layer, conducts heat away from touchscreen 120, functions as a heat sink, and/or otherwise reduces the amount of heat reaching touchscreen 120. In other embodiments, heat shield 813 is located on top of frame 801. In still further embodiments, heat shield 813 extends to cover all or a portion of power supply module 301 and/or main circuit board 401.

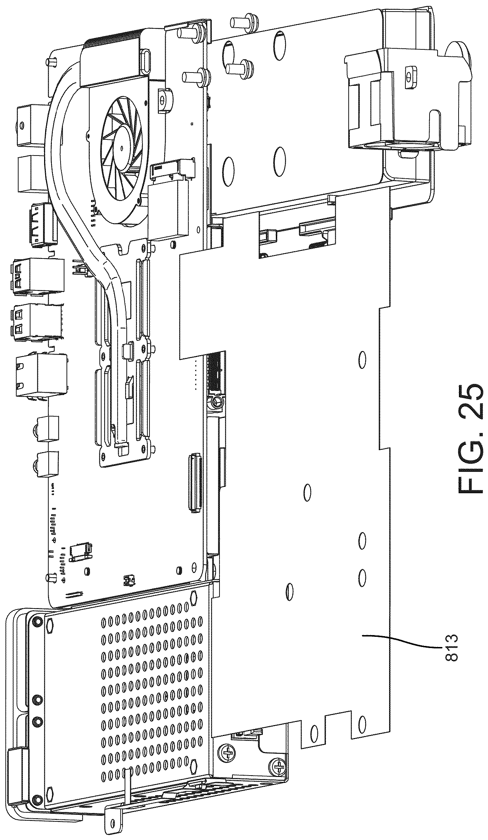

Referring now to FIG. 25, heat shield 813 primarily covers the space which may be occupied by ultrasound module 201 according to one embodiment. Heat shield 813 may shield components from heat generated only by ultrasound module 201. In other embodiments, heat shield 813 or a plurality of heat shields protect components from heat sources other than or in addition to ultrasound module 201.

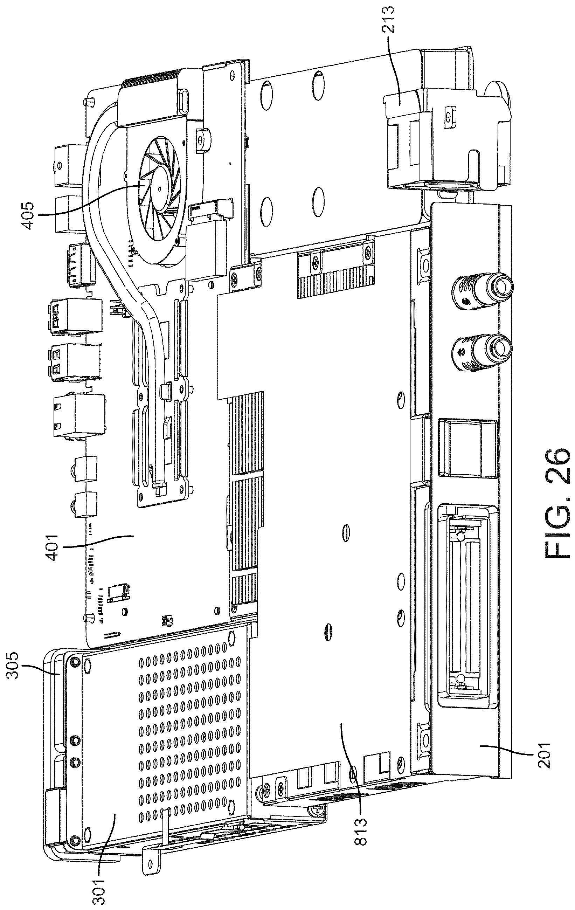

Referring now to FIG. 26, heat shield 813 may cover only a portion of ultrasound module 201 in some embodiments. For example, heat shield 813 may be configured to cover portions of ultrasound module 201 which contain major or primary sources of heat from within ultrasound module 201 (e.g., heat shield 813 may cover processors, integrated circuits, memory etc. but not cover portions of ultrasound module 201 in which connectors, buses, etc. are located). In other embodiments, heat shield 813 may cover all of ultrasound module 201.

In some embodiments, heat shield 813 is integrated into ultrasound module 201. For example, ultrasound modules 201 may include one or more heat shields 813 to prevent or mitigate heat transfer from ultrasound module 201 to other components of portable ultrasound system 100. In one embodiment, heat shield 813 is attached to an outer surface of ultrasound module 201. For example, heat shield 813 may be attached to or otherwise coupled to heat sink 207 of ultrasound module 201. Heat shield 813 may be attached using screws, nuts and bolts, adhesive, and/or other fasteners. In other embodiments, heat shield 813 is integral or internal to ultrasound module 201. For example, heat shield 813 may be included in or take the place of a heat sink 207. Alternatively and/or additionally, heat shield 813 may be included within ultrasound module 813. For example, heat shield 813 may be located within a housing of ultrasound module 201. In some embodiments, the housing of ultrasound module 201 may include heat shield 813 or be replaced by heat shield 813 or be made of a material with properties which allow it to function as a heat shield.

In some embodiments, the heat management system of portable ultrasound device includes one or more fans. Fans may provide a source of convection cooling for components of portable ultrasound device 100. The fans may provide the same or similar advantages described above with respect to the heat management system of portable ultrasound device 100. For example, fans may improve the effectiveness of air paths as relates to keeping components of portable ultrasound system 100 cool. Fans may facilitate or allow potable ultrasound system 100 to be packaged more compactly, allow ultrasound module 201 and other components to be located to facilitate removal and/or insertion of ultrasound module 201, keep other components cool, or otherwise provide an advantage to portable ultrasound system 100.

Referring now to FIG. 26, portable ultrasound system 100 includes three fans or three groups of fans in one embodiment. The fans may correspond to three air paths used for cooling components of portable ultrasound system 100. In some embodiments, each fan or group of fans corresponds to one module of portable ultrasound system 100. For example, ultrasound module fan 213 may provide cooling to ultrasound module 201. In some embodiments, ultrasound module fan 213 is configured to cool ultrasound module 201 in conjunction with an air path of ultrasound module 201 (e.g., by drawing air through the air path and/or over components of ultrasound module 201). Main circuit board module fan 401 may provide cooling to main circuit board module 401. Power supply fan 305 may provide cooling to power supply module 301. In one embodiment, power supply fan 305 includes two motor and fan assemblies. In further embodiments, portable ultrasound system 100 includes additional fans or groups of fans. Fans and/or groups of fans may be used to cool one or more components of portable ultrasound system 100. In some embodiments, one fan may cool components in multiple modules and/or other components of portable ultrasound system 100.

The present disclosure contemplates methods, systems, and program products on any machine-readable media for accomplishing various operations. The embodiments of the present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machineexecutable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general purpose computer, special purpose computer, or special purpose processing machines to perform a certain function or group of functions.

Although the figures may show a specific order of method steps, the order of the steps may differ from what is depicted. Also two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule based logic and other logic to accomplish the various connection steps, processing steps, comparison steps and decision step. The embodiments unrelated to claims are not according to the present disclosure and are present for illustration purposes only.

## Claims

1. A portable ultrasound system (100), comprising:
a user interface system comprising at least one display screen (120) and at least one user input device (110);
a main circuit board module (401) comprising a processing circuit (163) configured to perform general computing operations and configured to receive ultrasound imaging data from a removable ultrasound module (201);
a housing (150) containing the user interface system and the main circuit board module (401) and having an opening (803) configured to removably receive the removable ultrasound module (201); and
a connector (303) configured to form an electrical connection with a corresponding second connector of the removable ultrasound module (201) when the removable ultrasound module (201) is fully inserted into the portable ultrasound system (100) through the opening (803) of the housing (150), wherein the connector (303) comprises at least one guiding feature configured to align the connector (303) and the second connector as the removable ultrasound module (201) is inserted into the portable ultrasound system (100);
the portable ultrasound system (100) is configured to provide electrical power to the removable ultrasound module (201), and receive the ultrasound imaging data from the removable ultrasound module (201);
**characterized in that** the portable ultrasound system (100) further comprises a release lever (701), and the release lever (701) is configured to rotate around an axis parallel to a bottom face of the housing (150) during ejecting the removable ultrasound module (201) from the portable ultrasound system (100); the release lever (701) comprises an interlock mechanism that is controlled by the main circuit board module (401); and the interlock mechanism is normally engaged to prevent removal of the removable ultrasound module (201), and the main circuit board module (401) disengages the interlock mechanism when the main circuit board module (401) determines that it is safe to remove the removable ultrasound module (201).

2. The portable ultrasound system (100) of claim 1, wherein the removable ultrasound module (201) comprises hardware configured to generate ultrasound images and data and further comprises at least one ultrasound probe interface (185) configured to connect to an ultrasound probe (187).

3. The portable ultrasound system (100) of claim 1, wherein the connector is a fixed male connector (407) comprising:
contacts (423) configured to establish an electrical connection with second contacts included in the second connector;
guide slots (419) configured to receive guide shafts of the second connector such that the contacts and second contacts are aligned; and
a guide shaft (421) configured to be received by a guide slot of the second connector such that the contacts and second contacts are aligned.

4. The portable ultrasound system (100) of claim 1, further comprising:
a power supply system (301) comprising at least one battery; and
a female floating connector configured to form an electrical connection with a corresponding fixed male connector of the removable ultrasound module (201),
wherein the female floating connector is configured to have at least one degree of constrained freedom relative to the portable ultrasound system (100) as a whole.

5. The portable ultrasound system (100) of claim 1, wherein a lower portion of the release lever (701) is positioned such that the lower portion of the release lever (701) is not in contact with the removable ultrasound module (201) while the release lever (701) has not been rotated.

6. The portable ultrasound system (100) of claim 5, wherein the housing (150) comprises one or more shoulders (151) configured to guide, locate, and/or prevent over insertion of the removable ultrasound module (201); the release lever (701) is positioned relative to the removable ultrasound module (201) inserted in the housing (150); a position of the removable ultrasound module (201) is in turn defined by the one or more shoulders (151); the release lever (701) does not begin applying disconnecting force to the removable ultrasound module (201) until the release lever (701) has rotated thirty degrees due to a user input on an upper portion of the release lever (701); and the thirty degrees is defined by a positioning of the release lever (701) relative to the shoulders (151).

7. The portable ultrasound system (100) of claim 1, wherein the interlock mechanism is not a physical mechanism, the interlock mechanism is expressed in programming of the portable ultrasound system (100); the portable ultrasound system (100) is programmed such that an electromechanical system which disconnects and/or ejects the removable ultrasound module (201) is not activated unless the main circuit board module (401) determines that it is safe to disconnect the removable ultrasound module (201).

8. The portable ultrasound system (100) of claim 1, wherein the portable ultrasound system (100) further comprises a frame (801) configured to couple the user interface system and the processing circuit (163) to the housing and configured to support the removable ultrasound module (201); the frame comprises a floor portion (807) configured to support the removable ultrasound module (201) when inserted into the portable ultrasound system (100) and further configured to vertically align the removable ultrasound module (201) relative to the portable ultrasound system (100), a side portion (805) configured to laterally align the removable ultrasound module (201) relative to the portable ultrasound system (100), and a guide flange (809) coupled to the floor portion (807) and sloped upward towards the floor portion (807).

9. The portable ultrasound system (100) of claim 1, wherein the portable ultrasound system (100) further comprises the floating connector (303) of a power supply module (301) and a fixed connector (403) of the processing circuit (163) configured to form electrical connections with corresponding second connectors of the removable ultrasound module (201) when the removable ultrasound module (201) is fully inserted into the portable ultrasound system (100) through the opening (803) of the housing (150), the floating connector (303) and the fixed connector (403) comprise at least one guiding feature configured to align the floating connector (303) and the fixed connector (403) and the second connectors as the removable ultrasound module (201) is inserted into the portable ultrasound system (100), the fixed connector (403) and one second connector are configured for data transmission between the removable ultrasound module (201) and the processing circuit (163) of the portable ultrasound system (100), the floating connector (303) and another second connector are configured for power transmission from the power supply module (301) of the portable ultrasound system (100) to the removable ultrasound module (201), and the floating connector (303) and the fixed connector (403) are parallel for being configured to aligned with corresponding second connectors on the removable ultrasound module (201).

10. A system, **characterized by** comprising the portable ultrasound system (100) of any one of claims 1 to 9, and a removable ultrasound module (201) removably received in the housing (150).

## Patentansprüche

1. Ein tragbares Ultraschallsystem (100), das Folgendes umfasst:
Ein Benutzerschnittstellensystem, das mindestens einen Bildschirm (120) und mindestens eine Eingabeeinheit (110) umfasst;
Ein Hauptleiterplattenmodul (401), das einen Verarbeitungsschaltkreis (163) umfasst, der konfiguriert ist, um allgemeine Rechneroperationen auszuführen und konfiguriert ist, um ultraschallbildende Daten von einem abnehmbaren Ultraschallmodul (201) zu empfangen;
ein Gehäuse (150), das das Benutzerschnittstellensystem und das Hauptleiterplattenmodul (401) umfasst und eine Öffnung (803) aufweist, die konfiguriert ist, um das abnehmbare Ultraschallmodul (201) abnehmbar zu ermpfangen; und
ein Konnektor (303), der konfiguriert ist, um eine elektrische Verbindunng mit einem zweiten Konnektor des abnehmbaren Ultraschallmoduls (201) herzustellen, wenn das abnehmbare Ultraschallmodul (201) voll in das tragbare Ultraschallsystem (100) durch die Öffnung (801) des Gehäuses (150) eingefügt ist, wobei der Konnektor (303) mindestens eine Führungsfunktion aufweist, die konfiguriert ist, um den Konnektor(303) und den zweiten Konnektor auszurichten während das abnehmbare Ultraschallmodul (201) in das tragbare Ultraschallsystem (100) eingefügt wird;
wobei das tragbare Ultraschallsystem (100) konfiguriert ist, um das abnehmbare Ultraschallmodul (201) mit elektrischem Strom zu versorgen und die ultraschallbildenden Daten vom abnehmbaren Ultraschallmodul (201) zu erhalten;
**dadurch gekennzeichnet, dass** das tragbare Ultraschallsystem (100) weiter einen Auslösehebel (701) umfasst, und der Auslösehebel (701) konfiguriert ist, um um eine Achse zu drehen, die parallel zu einer Bodenfläche des Gehäuses (150) verläuft, während das abnehmbare Ultraschallmodul (201) vom tragbaren Ultraschallsystem (100) ausgestossen wird; wobei der Auslösehebel (701) einen Verriegelungsmechanismus umfasst, der durch das Hauptleiterplattenmodul (401) gesteuert wird; und wobei der Verriegelungsmechanismus normalerweise eingerastet ist, um das Abnehmen des abnehmbaren Ultraschallmoduls (201) zu vermeiden, und wobei das Hauptleiterplattenmodul (401) bestimmt, dass (ob) es sicher ist, das abnehmmare Ultraschallmodul (201) abzunehmen.

2. Das tragbare Ultraschallsystem (100) gemäss Anspruch 1, bei dem das abnehmbare Ultraschallmodul (201) Hardware umfasst, das konfiguriert ist, um Ultraschallbilder sowie Daten zu erzeugen, und weiter mindestens eine Ultraschallsondenschnittstelle (185) umfasst, das für den Anschluss an eine Ultraschallsonde (187) konfiguriert ist.

3. Das tragbare Ultraschallsystem (100) gemäss Anspruch 1, bei dem das Anschlussteil ein fester Stecker (407) ist, der Folgendes umfasst:
Kontakte (423), die konfiguriert sind, um eine elektrische Verbindung mit zweiten Kontakten herzustellen, die im zweiten Anschlussteil eingeschlossen sind;
Führungsnuten (419), die konfiguriert sind, um Führungswellen des zweiten Anschlussteils aufzunehmen, so dass die Kontakte und zweiten Kontakte ausgerichtet sind; und
eine Führungswelle (421), die konfiguriert ist, um in der Führungsnut des zweiten Anschlussteils aufgenommen zu werden, so dass die Kontakte und zweiten Kontakte ausgerichtet sind.

4. Das tragbare Ultraschallsystem (100) gemäss Anspruch 1, das weiter Folgendes umfasst:
Ein Energieversorgungssystem (301), das mindestens eine Batterie umfasst; und eine schwimmende Buchse umfasst, die konfiguriert ist, um eine elektrische Verbindung mit einem entsprechenden festen Stecker des abnehmbaren Ultraschallmoduls (201) zu bilden,
wobei die schwimmend Buchse konfiguriert ist, um mindestens einen Grad einer eingeschränkten Freiheit bezüglich des tragbaren Ultraschallsystems (100) als ein Ganzes zu haben.

5. Das tragbare Ultraschallsystem (100) gemäss Anspruch 1, bei dem ein unterer Teil des Auslösehebels (701) so positioniert ist, das der untere Teil des Auslösehebels (701) nicht mit dem abnehmbaren Ultraschallmodul (201 im Kontakt steht, während der Auslösehebel (701) nicht gedreht wurde.

6. Das tragbare Ultraschallsystem (100) gemäss Anspruch 5, bei dem das Gehäuse (150) eine oder mehrere Vorsprünge (151) umfasst, die konfiguriert sind, um übermässiges Einführen des abnehmbaren Ultraschallmoduls (201) zu leiten, lokalisieren und/oder vermeiden; wobei der Auslösehebel (701) mit Bezug auf das in das Gehäuse (150) eingefügte, abnehmbare Ultraschallmodul (201) positioniert ist; eine Position des abnehmbaren Ultraschallmoduls (201) wiederum durch den einen oder mehrere Vorsprünge (151) definiert ist; der Auslösehebel (701) nicht mit der Anwendung einer Unterbrechung der auf das abnehmbare Ultraschallmodul ausgeübten Kraft beginnt, bevor nicht der Auslösehebel (701) um dreissig Grad infolge einer Benutzereingabe an einerm oberen Teil des Auslösehebels (701) gedreht wurde; und die dreissig Grad durch ein Positionieren des Auslösehebels (701) bezüglich der Vorsprünge (151) definierus werden.

7. Das tragbare Ultraschallsystem (100) gemäss Anspruch 1, bei dem der Verriegelungsmechanismus nicht ein physischer Mechanismus ist, der Verriegelungsmechanismus in einer Programmierung des abnehmbaren Ultraschallsystems (100) dargestellt wird; das tragbare Ultraschallsystem (100) so programmiert wird, dass ein elektromechanisches System, das das abnehmbare Ultraschallmodul abschaltet und/oder ausstösst, nicht aktiviert ist, ausser wenn das Hauptleiterplattenmodul (401) feststellt, dass ein Abschalten des abnehmbaren Ultraschallmoduls (201) sicher ist.

8. Das tragbare Ultraschallsystem (100) gemäss Anspruch 1, wobei das tragbare Ultraschallsystem (100) weiter einen Rahmen (801) umfasst, der konfiguriert ist, um das Benutzerschnittstellensystem und den Schaltkreis (163) mit dem Gehäuse zu kuppeln, und konfiguriert ist, um das abnehmbare Ultraschallmodul (201) zu tragen; wobei der Rahmen eine Bodenteil (807) umfasst, der konfiguriert ist, um das abnehmbare Ultraschallmodul (201) zu tragen, wenn es in das tragbare Ultraschallsystem (100) eingesetzt wird, und iweiter konfiguriert ist, um senkrecht das abnehmbare Ultraschallmoldul (201) mit Bezug auf das tragbare Ultraschallsystem (100) auszurichten, wobei ein Seitenteil (805), konfiguriert ist, um seitlich das abnehmbare Ultraschallmodul (201) bezüglich des tragbaren Ultraschallsystems (100) auszurichten, und ein Führungsflansch (809), der am Bodentel (807) angekuppelt und nach oben in Richtung des Bodenteils (807) geneigt ist.

9. Das tragbare Ultraschallsystem (100) gemäss Anspruch 1, wobei das tragbare Ultraschallsystem (100) weiter den schwimmenden Konnektor (303) eines Energieversorgungsmoduls (301) und einen festen Konnektor (403) des Verarbeitungsschaltkreises (163) umfasst, der konfiguriert ist, um elektrische Anschlüsse mit entsprechenden zweiten Konnektoren des abnehmbaren Ultraschallmoduls (201) zu bilden, wenn das abehmbare Ultraschallmodul (201) voll in das tragbare Ultraschallsystem (100) durch die Öffnung (803) des Gehäuses (150) eingefügt ist, wobei der schwimmende Konnektor (303) und der feste Konnektor (403) mindestens eine Führungsfunktion umfassen, die konfiguriert ist, um den schwimmenden Konnektor (303) und den festen Konnektor (403) und die zweiten Konnektoren auszurichten, wenn das abnehmbare Ultraschallmodul (201) in das tragbare Ultraschallsystem (100) eingefügt wird, wobei der feste Konnektor (403) und ein zweiter Konnektor für Datenübertragung zwischen dem abnehmbaren Ultraschallmodul (201) und dem Verarbeitungsschaltkreis (163) des tragbaren Ultraschallsystems (100) konfiguriert ist, der schwimmende Konnektor (303) und ein anderer zweiter Konnektor für Energieübertragung vom Energieversorgungsmodul (301) des tragbaren Ultraschallsystems (100) zum abnehmbaren Ultraschallsystem (201) konfiguriert sind, und der schwimmende Konnektor (303) und feste Konnektor (403) parallel sind, um so konfiguriert zu sein, dass sie mit entsprechenden zweiten Konnektoren am abnehmbaren Ultraschallmodul (201) ausgerichtet werden können.

10. Ein System **dadurch gekennzeichnet dass** es das tragbare Ultraschallsystem (100) gemäss irgendeinem der Ansprüche 1 is 9 und ein abnehmbares Ultraschallmodul (201), das abnehmbar im Gehäuse (150) aufgenommen ist, umfasst.

## Revendications

1. Système portable à ultrasons (100), comprenant :
un système d'interface utilisateur comprenant au moins un écran d'affichage (120) et au moins un dispositif d'entrée utilisateur (110) ;
un module de carte de circuit imprimé principal (401) comprenant un circuit de traitement (163) configuré pour effectuer des opérations informatiques générales et configuré pour recevoir des données d'imagerie par ultrasons d'un module à ultrasons amovible (201) ;
un boîtier (150) contenant le système d'interface utilisateur et le module de carte de circuit imprimé principale (401) et pourvu d'une ouverture (803) configurée pour loger de manière amovible le module à ultrasons amovible (201) ; et
un connecteur (303) configuré pour former une connexion électrique avec un deuxième connecteur correspondant du module à ultrasons amovible (201) lorsque le module à ultrasons amovible (201) est entièrement inséré dans le système portable à ultrasons (100) à travers l'ouverture (803) du boîtier (150), dans lequel le connecteur (303) comprend au moins une fonction de guidage configurée pour aligner le connecteur (303) et le deuxième connecteur lorsque le module à ultrasons amovible (201) est inséré dans le système portable à ultrasons (100) ;
dans lequel le système portable à ultrasons (100) est configuré pour fournir une alimentation électrique au module à ultrasons amovible (201) et pour recevoir les données d'imagerie par ultrasons du module à ultrasons amovible (201) ;
**caractérisé en ce que** le système portable à ultrasons (100) comprend en outre un levier de déclenchement (701), et le levier de déclenchement (701) est configuré pour tourner autour d'un axe parallèle à la surface inférieure du boîtier (150) pendant l'éjection du module à ultrasons amovible (201) du système portable à ultrasons (100) ;
le levier de déclenchement (701) comprend un mécanisme de verrouillage qui est commandé par le module de carte de circuit imprimé principale (401) ; et le mécanisme de verrouillage est normalement engagé pour empêcher le retrait du module à ultrasons amovible (201). Le mécanisme de verrouillage est désengagé par le module de carte de circuit imprimé principal (401) lorsque celui-ci détermine qu'il est possible de retirer le module à ultrasons amovible (201) en toute sécurité.

2. Le système portable à ultrasons (100) selon la revendication 1, dans lequel le module amovible à ultrasons (201) comprend du matériel configuré pour générer des images et des données ultrasonores et comprend en outre au moins une interface de sonde ultrasonore (185) configurée pour se connecter à une sonde ultrasonore (187).

3. Le système portable à ultrasons (100) selon la revendication 1, dans lequel le connecteur est un connecteur mâle fixe (407) comprenant :
des contacts (423) configurés pour établir une connexion électrique avec des deuxièmes contacts inclus dans le deuxième connecteur ;
des rainures de guidage (419) configurées pour recevoir les tiges de guidage du deuxième connecteur de manière à ce que les contacts et les deuxièmes contacts soient alignés ; et
une tige de guidage (421) configurée pour être reçue par une rainure de guidage du deuxième connecteur de sorte que les contacts et les deuxièmes contacts soient alignés.

4. Le système portable à ultrasons (100) selon la revendication 1, comprenant en outre :
un système d'alimentation électrique (301) comprenant au moins une batterie ; et
un connecteur flottant femelle configuré pour former une connexion électrique avec un connecteur mâle fixe correspondant du module amovible à ultrasons (201),
dans lequel le connecteur flottant femelle est configuré pour avoir au moins un degré de liberté contraint par rapport à l'ensemble du système portable à ultrasons (100).

5. Le système portable à ultrasons (100) selon la revendication 1, dans lequel une partie inférieure du levier de déclenchement (701) est positionnée de telle sorte que la partie inférieure du levier de déclenchement (701) n'est pas en contact avec le module amovible à ultrasons (201) lorsque le levier de déclenchement (701) n'a pas été tourné.

6. Le système portable à ultrasons (100) selon la revendication 5, dans lequel le boîtier (150) comprend un ou plusieurs protubérances (151) configurés pour guider, localiser et/ou empêcher une insertion excessive du module amovible à ultrasons (201) ; le levier de déclenchement (701) est positionné par rapport au module amovible à ultrasons (201) inséré dans le boîtier (150) ; une position du module amovible à ultrasons (201) est à son tour définie par un ou plusieurs protubérances (151) ; le levier de déclenchement (701) ne commence pas à appliquer une force de déconnexion au module amovible à ultrasons (201) tant que le levier de déclenchement (701) n'a pas pivoté de trente degrés à la suite d'une action de l'utilisateur sur une portion supérieure du levier de déclenchement (701) ; les trente degrés sont définis par le positionnement du levier de déclenchement (701) par rapport aux protubérances (151).

7. Le système portable à ultrasons (100) selon la revendication 1, dans lequel le mécanisme de verrouillage n'est pas un mécanisme physique, le mécanisme de verrouillage est exprimé dans la programmation du système portable à ultrasons (100) ; le système portable à ultrasons (100) est programmé de telle sorte qu'un système électromécanique qui déconnecte et/ou éjecte le module amovible à ultrasons (201) n'est pas activé à moins que le module de la carte de circuit imprimé principale (401) ne détermine qu'il est sûr de déconnecter le module amovible à ultrasons (201).

8. Le système portable à ultrasons (100) selon la revendication 1, dans lequel le système portable à ultrasons (100) comprend en outre un cadre (801) configuré pour coupler le système d'interface utilisateur et le circuit de traitement (163) au boîtier, et configuré pour supporter le module amovible à ultrasons (201) ; le cadre comprend une portion de base (807) configurée pour supporter le module amovible à ultrasons (201) lorsqu'il est inséré dans le système portable à ultrasons (100) et configurée en outre pour aligner verticalement le module amovible à ultrasons (201) par rapport au système portable à ultrasons (100), une portion latérale (805) configurée pour aligner latéralement le module amovible à ultrasons (201) par rapport au système portable à ultrasons (100), et un rebord de guidage (809) couplé à la portion de base (807) et incliné vers le haut en direction de la portion de base (807).

9. Le système portable à ultrasons (100) selon la revendication 1, dans lequel le système portable à ultrasons (100) comprend en outre le connecteur flottant (303) d'un module d'alimentation électrique (301) et un connecteur fixe (403) du circuit de traitement (163) configurés pour former des connexions électriques avec les seconds connecteurs correspondants du module amovible à ultrasons (201) lorsque le module amovible à ultrasons (201) est entièrement inséré dans le système portable à ultrasons (100) à travers l'ouverture (803) du boîtier (150), le connecteur flottant (303) et le connecteur fixe (403) comprennent au moins une fonction de guidage configurée pour aligner le connecteur flottant (303) et le connecteur fixe (403) et les deuxièmes connecteurs lorsque le module amovible à ultrasons (201) est inséré dans le système portable à ultrasons (100), le connecteur fixe (403) et un deuxième connecteur sont configurés pour la transmission de données entre le module amovible à ultrasons (201) et le circuit de traitement (163) du système portable à ultrasons (100), le connecteur flottant (303) et un autre deuxième connecteur sont configurés pour la transmission d'énergie provenant du module d'alimentation (301) du système portable à ultrasons (100) vers le module amovible à ultrasons (201), et le connecteur flottant (303) et le connecteur fixe (403) sont parallèles de manière à être configurés pour s'aligner avec les deuxièmes connecteurs correspondants sur le module amovible à ultrasons (201).

10. Un système, **caractérisé en ce qu'**il comprend le système portable à ultrasons (100) selon une quelconque des revendications 1 à 9, et un module à ultrasons amovible (201) logé de manière amovible dans le boîtier (150).
